# EUROPEAN PATENT APPLICATION

(11) **EP 2 316 474 A1**
(43) Date of publication of application: **04.05.2011**
(21) Application number: 10013145.7
(22) Date of filing: 31.07.2002
(51) Int. Cl.: A61K 38/43, A61K 38/48, C12N 15/52, C12N 15/57, C12Q 1/37

(54) **Pregnancy-related enzyme PC 5/6 antagonists**

(30) Priority: 31.07.2001 AU PR673001
(62) Divisional of application: 02750655.9
(71) Applicant: Prince Henry's Institute of Medical Research, Clayton VIC 3168 (AU)
(72) Inventor: Nie, Guiying, Chadstone Victoria 3148 (AU); Salamonsen, Lois Adrienne, Kew Victoria 3101 (AU); Findlay, John Kerr, Hawthorn Victoria 3122 (AU)
(74) Representative: Webber, Philip Michael

(57) **Abstract**

This invention relates to the role of the enzyme proprotein convertase 5/6 in pregnancy, and in particular to the detection or modulation of proprotein convertase 5/6 and its isoforms in the uterus. This enzyme is useful in the control of fertility, the monitoring of early pregnancy, and for the detection of uterine receptivity. The invention also relates to methods of screening for compounds which have the ability to modulate the activity or expression of proprotein convertase 5/6, which may be useful in regulating fertility in mammals.
Novel forms of proprotein convertase 5/6 are also disclosed and claimed.

## Description

This invention relates to the role of the enzyme proprotein convertase 5/6 in pregnancy.

### BACKGROUND

Embryo implantation, the process by which the blastocyst attaches to and implants in the uterus, leads to the establishment of an intimate relationship between the embryo and the endometrium. Implantation is one of the most important limiting factors in establishing a successful pregnancy. It is a complex process involving active interactions between the blastocyst and the uterus. The uterus must undergo dramatic morphological and physiological changes to transform itself from a non-receptive to a receptive state. This differentiation process is primarily mediated by the coordinated effects of the ovarian hormones, which act through their intracellular receptors to regulate gene expression, and hence to influence cellular proliferation and differentiation.

While the details of the exact molecular events occurring in the uterus during this differentiation process towards receptivity are still unknown, in principle it can be predicted that a unique set of genes is up- or down-regulated in a temporally and spatially specific manner. Indeed, induction of specific genes in the uterus during the peri-implantation period, including those encoding some growth factors and cytokines, has been reported. However, given the complexity and the as-yet imprecisely defined molecular mechanisms of the process, many other molecules critical for implantation are still unidentified.

We hypothesised that the proliferation and differentiation of endometrial cells at this time is associated with up- or down-regulation of a number of genes, many of which are still unknown (Nie et al, 1997).

### SUMMARY

We have examined the uterine expression of PC 5/6 during early pregnancy in the mouse, and have demonstrated for the first time that this proprotein convertase is specifically upregulated in the mouse uterus at the sites of embryo attachment, and that its expression is restricted to the decidualized stromal cells. We have also identified an isoform of PC 5/6, which appears to be specifically expressed in implantation sites of the uterus during the embryo's implantation period. We have also detected PC 5/6 expression in human endometrium.

Proprotein convertase 5/6 is believed to be useful in promoting the implantation of the fertilized egg in the uterus, development of the embryo and maintenance of pregnancy. Therefore modulation of the activity of this enzyme may be used to promote or to inhibit fertility. It is also contemplated that modulation of PC 5/6 activity could be used to prevent the establishment of a pregnancy, ie that this could be used as a contraceptive.

A first aspect provides a method of detecting the fertile period in a female mammal, comprising the step of measuring the activity of or detecting the presence of proprotein convertase 5/6 in a biological sample from the mammal.

A second aspect provides a method of detecting whether the uterus of a female mammal is in a receptive state, comprising the step of detecting the presence or absence of PC 5/6, or its presence in increased amounts at a particular stage of the cycle compared with another stage, whereby the presence of or an increase in PC 5/6 means that the uterus is in a receptive state.

The presence or absence of PC 5/6 may be assessed by detecting the activity of the enzyme itself, eg. using a specific antibody directed against the enzyme, or by detecting nucleic acid, encoding the enzyme, for example using PCR or in *in situ* hybridization.

A third aspect provides a method of inhibiting the conversion of a non-receptive state of the uterus of a female mammal to a receptive state, comprising the step of administering an antagonist of PC 5/6 to a female mammal in need of such treatment.

A fourth aspect provides a method of inhibiting fertility, including but not limited to inhibiting uterine receptivity and/or embryo implantation, in a female mammal, comprising the step of administering an antagonist of PC 5/6 to a female mammal in need of such treatment A fifth aspect provides a method of screening for compounds which have the ability to modulate the activity of proprotein convertase 5/6, comprising the step of assessing the ability of a candidate compound to increase or decrease proprotein convertase 5/6 activity. The person skilled in the art will appreciate that a wide range of activities can be measured, for example stimulation of gene expression, stimulation of activation of PC 5/6 or inhibition of degradation by PC 5/6.

A sixth aspect provides a method of identifying molecules necessary for implantation, comprising the step of testing a candidate molecule for the ability to promote the conversion of protein precursors cleavable by PC 5/6 into mature proteins.

The biological sample may be uterine tissue, washings from the uterine cavity, or another biological fluid, such as blood, plasma, serum or saliva.

In all embodiments the PC 5/6 may have:
(a) the nucleic acid sequence set out in SEQ ID NO: 1 or SEQ ID NO: 2, or its human homologue (SEQ ID NO: 3);
(b) a nucleic acid molecule which is able to hybridise under at least moderately stringent conditions to (a); and
(c) a nucleic acid molecule which has at least 75% sequence identity to (a).

It will be clearly understood that for the purposes of the invention different isoforms of PC 5/6 may be suitable. Preferably in all aspects of the invention, an isoform of PC 5/6 which is specifically expressed in implantation sites of the uterus during embryo implantation is used. In view of the crucial role in implantation indicated by the results reported herein, it is contemplated that antagonists of PC 5/6 will be useful as contraceptives, and that agonists of PC 5/6 will be useful as agents for promoting fertility or for supporting at least the early phases of pregnancy. It is further contemplated that suitable antagonists of PC 5/6 include, but are not limited to, antibodies and anti-sense nucleic acids. For example, an inhibitory antisense 17mer oligonucleotide is disclosed in International Patent Application No. PCT/CA97/00535 (WO98/04686) by Day et al.

As used herein, the term "receptive" refers to a state of the uterine lining which will allow an embryo to implant, whereas a "non-receptive" state refers to a state in which attachment and implantation are impaired. This can include a state known as "pre-receptive".

The term "PC 5/6 antagonist" or "antagonist" refers to a substance which opposes or interferes with a functional activity of PC 5/6.

For the purposes of this specification it will be clearly understood that the word "comprising" means "including but not limited to", and that the word "comprises" has a corresponding meaning.

### BRIEF DESCRIPTION OF THE FIGURES

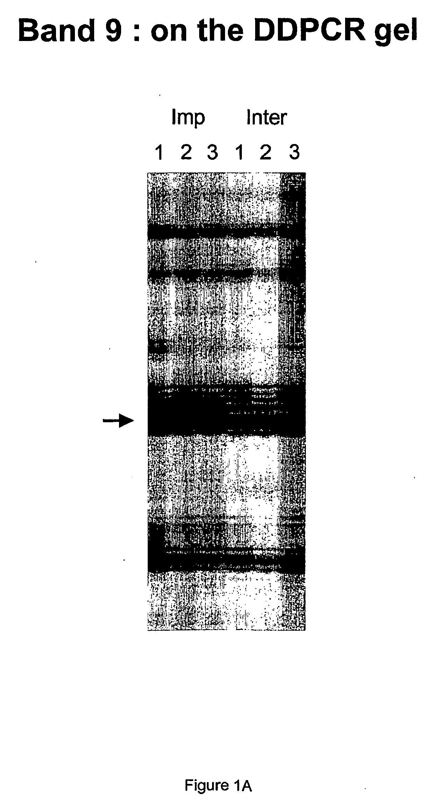

Figure 1A shows the results of RNA differential display analysis (DDPCR) of pregnant mouse uterus. The expression pattern of band 9 to be identified as PC 5/6 on the DDPCR gel is indicated by the arrow, showing much stronger intensities in implantation sites (Imp) compared to interimplantation sites (Inter) in three different mice: lane 1, animal 1; lane 2, animal 2 and lane 3, animal 3.

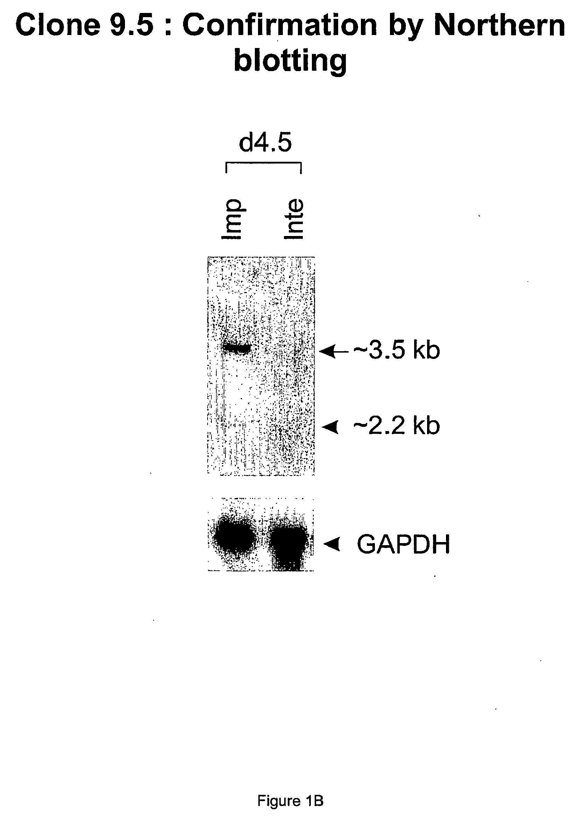

Figure 1B shows the results of Northern blot analysis of mRNA detected by the cDNA extracted from band 9 of the DDPCR gel and used as a probe. Total RNA (15µg) was isolated from implantation (Imp) and interimplantation sites (Inter) of day 4.5 pregnant mice. The top panel shows multiple bands, mainly at -2.2 kb and -3.5 kb positions; the lower panel shows the signal detected by the GAPDH probe on the same membrane as in the top panel.

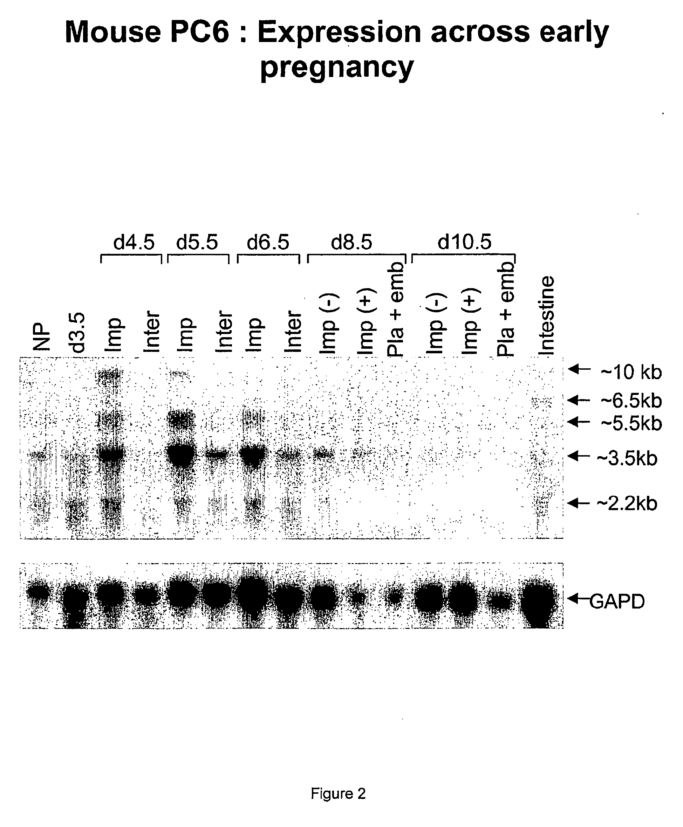

Figure 2 shows the results of Northern blot analysis of mRNA detected by the MUSPC6-fragB cDNA in the mouse uterus during early pregnancy and in the intestine. Total RNA (15µg) was isolated from whole uterus of non-pregnant mice at estrus (NP) and 3.5 day pregnant (d3.5) mice, and from implantation sites (Imp) and interimplantation sites (Inter) on day (d) 4.5, 5.5, 6.5, 8.5 and 10.5 of pregnancy. On day 8.5 and 10.5, three types of tissue were sampled:
   (1) the entire implantation unit containing the uterine implantation site, embryo and placenta [Imp (+)],
   (2) uterine implantation site tissue without embryo and placenta [Imp(-)], and
   (3) embryo and placenta sampled together (Emb +P1).
   The top panel shows the multiple transcripts (2.2 kb, 3.5 kb, 5.5 kb, 6.5 kb and 10 kb) detected by this cDNA, and the lower panel shows the signal detected by the glyceraldehyde-3-phosphate dehydrogenase (GAPDH) probe on the same membrane.
Figure 3 shows the results of Northern blot analysis of total RNA (15µg) isolated from whole uterus of non-pregnant mice at metestrus (met), diestrus (die), proestrus (pro) and estrus (est). One typical cycle is shown. The top panel shows the signals detected by the MUSPC6-fragB cDNA; the lower panel shows that detected by the GAPDH probe on the same membrane.
Figure 4 shows the results of Northern blot analysis of total RNA (15µg) isolated from whole uterus of non-pregnant mice at estrus (NP), from implantation sites (Imp) and interimplantation sites (Inter) of day (d) 4.5 pregnant mice, and from whole uterus of 4.5 pseudopregnant (Pseudo preg) mice. Day 0 is taken as the day of finding the vaginal plug resulting from copulation. The top panel shows signals detected by the MUSPC6-fragB cDNA; the lower panel shows that detected by the GAPDH probe on the same membrane.
Figure 5 shows the results of Northern blot analysis of the tissue specificity of the MUSPC6 mRNA. Total RNA (15µg) was isolated from mouse muscle, whole brain, kidney, spleen, heart, testis, ovary, implantation site on day 5.5 of pregnancy (d5.5-Imp), intestine, lung, liver and placenta. The top panel shows the signals detected by MUSPC6-fragB cDNA, and the lower panel shows the signal detected by ribosomal 18s RNA probe on the same membrane.

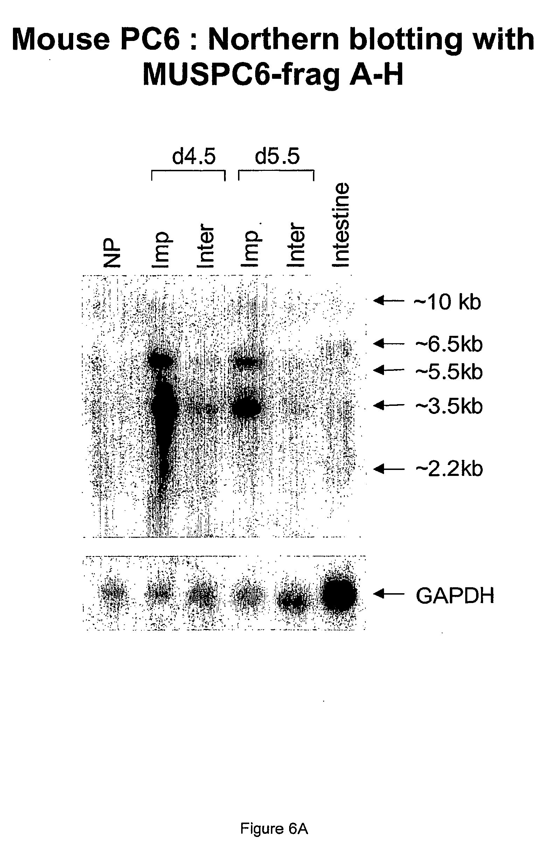

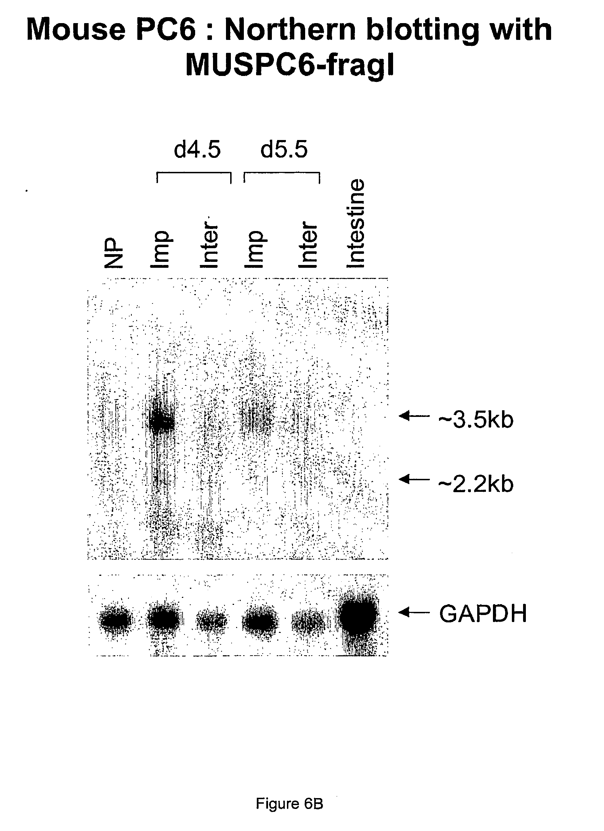

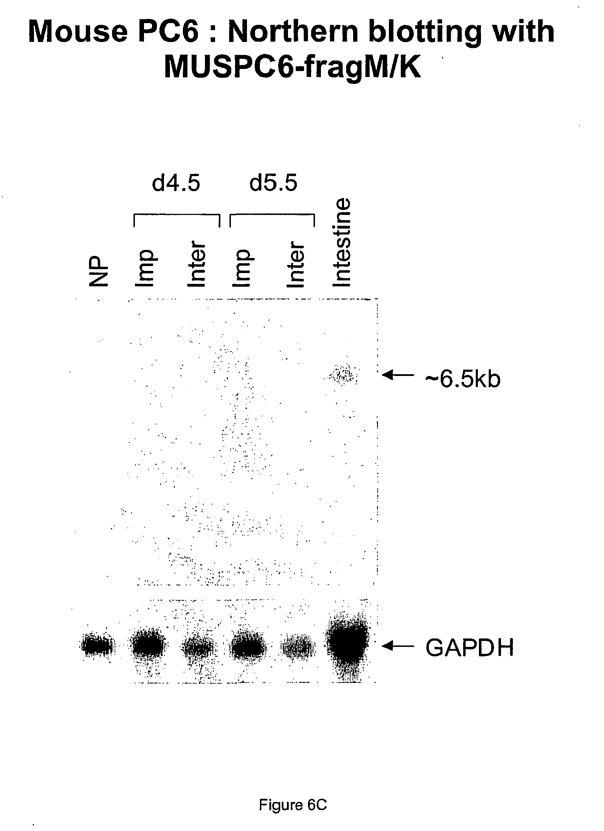

Figure 6 shows the results of Northern blot analysis performed to compare the mRNA transcript profiles between the uterus and the intestine. Total RNA (15µg) was isolated from whole uterus of non-pregnant mice at estrus (NP), and from implantation sites (Imp) and interimplantation sites (Inter) on day (d) 4.5 and 5.5 of pregnancy. Total RNA (25µg) was also isolated from the mouse intestine. The top panels show the mRNA patterns detected by the cDNA fragments representing the different domains of the MUSPC6 proteins, and the lower panel shows the signal detected by a GAPDH probe on the same membrane. (A) The multiple transcripts detected by MUSPC6-fragA/DA/DB/G/H cDNA; (B) The 3.5 kb transcript detected by MUSPC6-fragl; (C) The 6.5 kb transcript detected by MUSPC6-fragK/M.
Figure 7 shows the results of Northern blot analysis of the tissue specificity of the MUSPC6B mRNA. Total RNA (15µg) was isolated from mouse muscle, whole brain, kidney, spleen, heart, ovary, testis, liver, lung, intestine, and placenta, and from implantation sites (Imp) and interimplantation (Inter) sites on day 4.5 of pregnancy. The top panel shows the 6.5 kb transcript detected by MUSPC6-fragK/M cDNA, and the lower panel shows the signal detected by ribosomal 18s RNA probe on the same membrane.
Figure 8 shows the results of Southern blot analysis of MUSPC6 DNA in the mouse. Genomic DNA was isolated from non-pregnant mouse uterus, and 10µg was digested with the following four restriction enzymes: BamH1, EcoR1, HindIII and Bg1II probe with radio-labeled MUSPC6-fragH cDNA.
Figure 9 shows the results of in situ hybridisation of MUSPC6 mRNA in a section of mouse uterus at an implantation site on day 4.5 of pregnancy. Artificially decidualized uterus was also tested.
Figure 10 shows an MTE array (Clontech) demonstrating expression of PC6 mRNA in a range of human tissues, including the uterus, heart, gastrointestinal tract, kidney, thymus, lung, placenta, testis and ovary. The probe was a human PC6 cDNA.
Figure 11 shows the results of Northern blot analysis of a range of human tissues using a human PC6 cDNA probe. Total RNA (10µg) was applied to each lane. The probe was a human PC6 cDNA.
Figure 12 shows results of RT-PCR for PC6 on human endometrial samples taken across the normal menstrual cycle.
Figure 13 shows results of RT-PCR of human PC6 in early pregnant human tissue (first trimester decidua and placenta), heart, perimenopausal ovary, post-menopausal ovary, and term placenta.
Figure 14 shows results of real-time quantitative RT-PCR analysis of human PC6 mRNA from human endometrial cancer tissues (stages 1-3).

### DETAILED DESCRIPTION

PC 5/6 may be used as an immunogen to generate anti-PC 5/6 antibodies. Such antibodies, which specifically bind to PC 5/6, are useful in assays for PC 5/6, such as radioimmunoassay, enzyme-linked immunoassay, or competitive-type receptor binding assay or radioreceptor assay, as well as in affinity purification techniques. The anti-PC 5/6 antibody should preferably bind PC 5/6 with an affinity of at least about 10⁶ L/mole, and more preferably at least about 10⁷ Umole.

Polyclonal antibodies directed toward PC 5/6 are generally raised in animals by multiple subcutaneous or intraperitoneal injections of PC 5/6 and an adjuvant. If necessary, immunogenicity may be increased by conjugating PC 5/6 or a peptide fragment thereof to a carrier protein which is immunogenic in the species to be immunized, such as keyhole limpet hemocyanin, serum albumin, bovine thyroglobulin, soybean trypsin inhibitor, or pertussis toxin, using a bifunctional or derivatizing agent, for example, maleimidobenzoyl sulfosuccinimide ester (conjugation through cysteine residues), N-hydroxysuccinimide (conjugation through lysine residues), glutaraldehyde, succinic anhydride, SOCI2, or R¹N = C = NR, where R and R¹ are different alkyl groups.

It will be appreciated that a fragment or derivative of PC 5/6 which retains the ability to elicit anti-PC 5/6 antibody may alternatively be used as the immunogen.

Monoclonal antibodies directed toward PC 5/6 are produced using any method which provides for the production of antibody molecules by continuous cell lines in culture. The modifier "monoclonal" indicates the character of the antibody as being obtained from a substantially homogeneous population of antibodies, and is not to be construed as requiring production of the antibody by any particular method.

The monoclonal antibodies of the invention specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (Cabilly et al., U.S. Patent No. 4,816,567; Morrison et al 1984).

In a preferred embodiment, the chimeric anti-PC 5/6 antibody is a "humanized" antibody. Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain.

For diagnostic applications, anti-PC 5/6 antibodies typically will be labeled with a detectable moiety. The detectable moiety can be any one which is capable of producing, either directly or indirectly, a detectable signal. For example, the detectable moiety may be a radioisotope, such as ³H, ¹⁴C, ³²P, ³³P, ³⁵S, or ¹²⁵I , a fluorescent or chemiluminescent compound, such as fluorescein isothiocyanate, rhodamine, or luciferin; radioactive isotopic labels, such as ¹²⁵I, ³²P ³³P, ¹⁴C, or ³H, or an enzyme, such as alkaline phosphatase, beta-galactosidase or horseradish peroxidase.

The anti-PC 5/6 antibodies may be employed in any known assay method, such as competitive binding assays, direct and indirect sandwich assays, and immunoprecipitation assays.

Competitive binding assays rely on the ability of a labeled standard (e.g., PC 5/6 or an immunologically reactive portion thereof) to compete with the test sample analyte (PC 5/6) for binding with a limited amount of antibody. The amount of PC 5/6 in the test sample is inversely proportional to the amount of standard that becomes bound to the antibodies. To facilitate determining the amount of standard that becomes bound, the antibodies generally are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex (U.S. Patent No. 4,376,110). The second antibody may itself be labeled with a detectable moiety (direct sandwich assays) or may be measured using an anti-immunoglobulin antibody which is labeled with a detectable moiety (indirect sandwich assay). For example, one type of sandwich assay is an ELISA assay, in which case the detectable moiety is an enzyme.

Neutralizing anti-PC 5/6 antibodies are useful as antagonists of PC 5/6. The term "neutralizing anti-PC 5/6 antibody" as used herein refers to an antibody which is capable of specifically binding to PC 5/6, and which is capable of substantially inhibiting or eliminating the functional activity of PC 5/6 in vivo or in vitro. Typically a neutralizing antibody will inhibit the functional activity of PC 5/6 by at least about 50%, and preferably greater than 80%, as determined, for example, by an in vitro receptor binding assay.

PC 5/6 is believed to be useful in promoting the implantation of the fertilized egg, development of the placenta and the embryo, and maintenance of pregnancy. Accordingly, PC 5/6 may provide a potential target for contraception. It is also contemplated that it may be implicated in other conditions, such as cancers, and in particular cancers of the reproductive system, such as endometrial cancer.

PC 5/6 may be formulated with other ingredients such as carriers and/or adjuvants, e.g. albumin, nonionic surfactants and other emulsifiers. There are no limitations on the nature of such other ingredients, except that they must be pharmaceutically acceptable, efficacious for their intended administration, and cannot degrade the activity of the active ingredients of the compositions. Suitable adjuvants include collagen or hyaluronic acid preparations, fibronectin, factor XIII, or other proteins or substances designed to stabilize or otherwise enhance the active therapeutic ingredient(s).

Animals or humans may be treated in accordance with this invention. It is possible but not preferred to treat an animal of one species with PC 5/6 of another species.

A number of inhibitors of proprotein convertases have been described. These include alpha₁-antitrypsin Portland (Jean et al, 1998) and N-terminal prosegments of the PCs which inhibit the parent enzyme (PC1/3, Boudreault et al, 1998; Furin and PC7, Zhong et al, 1999). Although these are either non-specific or not shown for PC 5/6, similar inhibitors could be prepared which would be useful for the applications described herein.

PC 5/6 and PC 5/6 antagonists to be used for in vivo administration must be sterile. This is readily accomplished by filtration of a solution of PC 5/6 or anti-PC 5/6 antibody through sterile filtration membranes. Thereafter, the filtered solution may be placed into a container having a sterile access port, for example, an intravenous solution bag or vial having a stopper pierceable by a hypodermic injection needle. The filtered solution also may be lyophilized to produce sterile PC 5/6 or anti-PC 5/6 antibody in a powder form.

Methods for administering PC 5/6 and PC 5/6 antagonists, such as synthetic inhibitors, in vivo include injection or infusion by intravenous, intraperitoneal, intracerebral, intrathecal, intramuscular, intraocular, intraarterial, intravaginal, intrauterine, intracervical, or intralesional routes, and by means of sustained-release formulations, including suppositories.

Sustained-release formulations generally consist of PC 5/6 or PC 5/6 antagonists and a matrix from which the PC 5/6 or PC 5/6 antagonists are released over some period of time. Suitable matrices include semipermeable polymer matrices in the form of shaped articles, for example, membranes, fibers, or microcapsules. Sustained release matrices may comprise polyesters, hydrogels, polylactides, U.S. Patent No. 3,773,919, copolymers of L-glutamic acid and gamma ethyl-L-glutamate, Sidman et al., 1983, poly (2-hydroxyethyl-methacrylate), or ethylene vinyl acetate, Langer et al., 1981 & 1982. In one embodiment of the invention, the therapeutic formulation comprises PC 5/6 or a PC 5/6 antagonist entrapped within or complexed with liposomes. For example, PC 5/6 covalently joined to a glycophosphatidyl-inositol moiety may be used to form a liposome comprising PC 5/6.

An effective amount of PC 5/6 or PC 5/6 antagonist, e.g., anti-PC 5/6 antibody, to be employed therapeutically will depend upon the therapeutic objectives, the route of administration, and the condition of the patient. Accordingly, it will be necessary for the therapist to titrate the dosage and modify the route of administration as required to obtain the optimal therapeutic effect. A typical daily dosage might range from about 1 µg/kg to up to 100 mg/kg or more, depending on the factors mentioned above. Much lower doses may be possible if the agent is administered locally. Where possible, it is desirable to determine appropriate dosage ranges first in vitro, for example, using assays for serine protease activity or specific PC 5/6 activity which are known in the art, and then in suitable animal models, from which dosage ranges for human patients may be extrapolated.

For example, the dose of a protein PC 5/6 antagonist, particularly an antibody, can be about 0.1 mg to about 500 mg, typically about 1.0 mg to about 300 mg, more typically about 25 mg to about 100 mg. The administration frequency can be appropriately selected depending upon the condition to be treated and the dosage form for the desired therapeutic effects.

The mammal may be a human, or may be a domestic or companion animal. While it is particularly contemplated that the compounds of the invention are suitable for use in medical treatment of humans, they are also applicable to veterinary treatment, including treatment of companion animals such as dogs and cats, and domestic animals such as horses, cattle and sheep, or zoo animals or feral mammals such as non-human primates, felids, canids, bovids, and ungulates.

Methods and pharmaceutical carriers for preparation of pharmaceutical compositions are well known in the art, as set out in textbooks such as Remington's Pharmaceutical Sciences, 20th Edition, Williams and Wilkins, Pennsylvania, USA.

The compounds and compositions of the invention may be administered by any suitable route, and the person skilled in the art will readily be able to determine the most suitable route and dose for the condition to be treated. Dosage will be at the discretion of the attendant physician or veterinarian, and will depend on the nature and state of the condition to be treated, the age and general state of health of the subject to be treated, the route of administration, and any previous treatment which may have been administered.

The carrier or diluent, and other excipients, will depend on the route of administration, and again the person skilled in the art will readily be able to determine the most suitable formulation for each particular case.

As used herein, the term "therapeutically effective amount" means an amount of a compound of the present invention effective to yield a desired therapeutic response, for example to prevent or treat a disease which is susceptible to treatment by administration of a pharmaceutically-active agent.

The specific "therapeutically effective amount" will of course vary with such factors as the particular condition being treated, the physical condition and clinical history of the subject, the type of animal being treated, the duration of the treatment, the nature of concurrent therapy (if any), and the specific formulations employed and the structure of the compound or its derivatives.

As used herein, a "pharmaceutical carrier" is a pharmaceutically acceptable solvent, suspending agent, excipient or vehicle for delivering the compound of formula I and/or pharmaceutically-active agent to the subject. The carrier may be liquid or solid, and is selected with the planned manner of administration in mind.

The compounds of the invention may be administered orally, topically, or parenterally in dosage unit formulations containing conventional non-toxic pharmaceutically acceptable carriers, adjuvants, and vehicles. The term parenteral as used herein includes subcutaneous, intravenous, intramuscular, intrathecal, intracranial, injection or infusion techniques.

Generally, the terms "treating", "treatment" and the like are used herein to mean affecting a subject, tissue or cell to obtain a desired pharmacological and/or physiological effect. The effect may be prophylactic in terms of completely or partially preventing a disease or sign or symptom thereof, and/or may be therapeutic in terms of a partial or complete cure of a disease. "Treating" as used herein covers any treatment of, or prevention of disease in a vertebrate, a mammal, particularly a human, and includes: preventing the disease from occurring in a subject who may be predisposed to the disease, but has not yet been diagnosed as having it; inhibiting the disease, ie., arresting its development; or relieving or ameliorating the effects of the disease, ie., causing regression of the effects of the disease. The invention will now be described in detail by way of reference only to the following nonlimiting examples and drawings.

### MATERIALS AND METHODS

### Animals and Tissue Preparation

Swiss outbred mice were housed and handled according to the Monash University animal ethics guidelines on the care and use of laboratory animals. All experimentation was approved by the Institutional Animal Ethics Committee at the Monash Medical Centre. Adult female mice (6-8 weeks old) were mated with fertile males of the same strain to produce normal pregnant animals, or mated with vasectomized males to produce pseudopregnant mice. The morning of finding a vaginal plug was designated as day 0 of pregnancy. Uterine tissues were collected from non-pregnant mice, or from pregnant mice on days 3-11. A selection of other mouse organs was also collected from non-pregnant mice. Tissues were snap-frozen in liquid nitrogen for Northern analysis, or fixed in 4% buffered formalin (pH 7.6) for in situ hybridisation.

For non-pregnant and day 3.5 pregnant mice, the entire uterus was collected. For day 4.5 pregnant mice, implantation sites were visualised by intravenous injections of a Chicago Blue dye solution (1% in saline, 0.1 ml/mouse) into the tail vein 5 min before killing the animals; implantation sites were separated from interimplantation sites, and both sites were retained. For pregnant mice on day 5.5 onwards, implantation and interimplantation sites were visualized without dye injection.

For non-pregnant mice, the uterus was also collected from different stages of the estrous cycle: metestrus, diestrus, proestrus and estrus. The stages of the cycle were determined by analysis of vaginal smears. For ovarian hormone treatments, the animals were first ovariectomized under anaesthesia with avertin, without regard to the stage of the estrous cycle.

### Example 1: RNA Differential Display (DDPCR) and isolation of cDNAs

DDPCR was performed as previously described (Nie et al, 2000b), and as described originally by Liang and Pardee (1992 & 1993).

Total RNA was extracted from pools of implantation or interimplantation site tissues of mice by acid guanidinium thiocyanate-phenol-chloloroform extraction (Chomczynski and Sacchi, 1987). The RNA was then treated with RNase-free DNase in the presence of placental RNase inhibitor to remove any contaminating DNA. The amount of RNA in the final preparation was determined spectrophotometrically, and the RNA quality was evaluated by the ratio of OD260 over OD280 (>1.8). DNA-free RNA (1(g) from the implantation and interimplantation sites of three animals was used as the template for the first-strand cDNA synthesis in a 20 µl reaction mixture in the presence of 20 µM dNTPs, 50 µM oligo-dT anchored primers (one of T12MG, T12MC, T12MA and T12MT), as shown in Table 1, 10 mM DTT, 10 U RNasin ribonuclease inhibitor (Promega, Madison, WI), 25 U AMV reverse transcriptase (Boehringer Mannheim, Nunawading, Victoria, Australia), and the cDNA synthesis buffer (Boehringer Mannheim). The synthesised cDNA was then amplified by PCR in 20 µl with the following components: 2µl of cDNA, 1XPCR buffer (10 mM Tris-HCI, 1.5 mM MgC1₂, 50 mM KC1, pH 8.3), 10 µM dNTPs, 10 picomoles of one random decamer (Table 1, Operon 10-mer kit A; Operon, Alameda, CA), 50 picomoles of one oligo-dT anchored primer (as used in cDNA synthesis), 2 (Ci of ³³P-dATP (Du Pont Ltd., North Sydney, Australia) and 1 U of Taq DNA polymerase (Boehringer Mannheim). The PCR was performed in a Hybaid OmniGene PCR system (Hybaid Ltd, Teddington, Middlesex, UK) with the following conditions: initial denaturation at 94°C for 5 min; then 40 cycles of denaturation at 94°C for 30 sec, primer annealing at 39°C for 2 min, and a final extension at 72°C for 30 sec; and a final extension at 72°C for 10 min. The subsequent PCR products (4 µl) were run on 6% high-resolution polyacrylamide/urea gel (Liang & Pardee, 1992) and visualised by autoradiography. The majority of amplified fragments were shared between the implantation and interimplantation sites. Bands unique to implantation sites compared to interimplantation sites were identified as of interest. The 80 PCR primer combinations (20 random 10 mers combined with 4 oligo-dT anchored primers) used in the initial DDPCR analysis are shown in Table 1.

The candidate bands obtained from the DDPCR analysis were excised from the dried sequencing gel, incubated with 100 µl H₂O and 2 drops of oil at 100°C for 15 min, followed by room temperature incubation for 1 hr, and then the mixture was vigorously vortexed before centrifugation for 5 min to remove debris and obtain the cDNA. The eluted cDNAs were reamplified by PCR using the conditions described above. The differential display pattern was further confirmed by Northern blotting analysis using as probes the reamplified PCR products, which were generated by random hexamer labelling.

**Table 1**

| The 80 (4x20) primer combinations used in DDPCR | | | | | | | |
|---|---|---|---|---|---|---|---|
| 3' primers: Oligo-(dT) anchored primers, custom-made | | | | | | | |
| Primer | Code | Sequence | | | | SEQ ID NO. | |
| 1 | T12MA | TTTTTTTTTTTT(G,A,C)A | | | | 7 | |
| 2 | T12MC | TTTTTTTTTTTT(G,A,C)C | | | | 8 | |
| 3 | T12MG | TTTTTTTTTTTT(G,A,C)G | | | | 9 | |
| 4 | T12MT | TTTTTTTTTTTT(G,A,C)T | | | | 10 | |
| | | | | | | | |

| 5' Primers: 10 mers, from OPERON | | | | | | | |
|---|---|---|---|---|---|---|---|
| Primer | Code | Sequence | SEQ ID NO. | Primer | Code | Sequence | SEQ ID NO. |
| 1 | OPA-01 | CAGGCCCTTC | 11 | 11 | OPA-11 | CAATCGCCGT | 21 |
| 2 | OPA-02 | TGCCGAGCTG | 12 | 12 | OPA-12 | TCGGCGATAG | 22 |
| 3 | OPA-03 | AGTCAGCCAC | 13 | 13 | OPA-13 | CAGCACCCAC | 23 |
| 4 | OPA-04 | AATCGGGCTG | 14 | 14 | OPA-14 | TCTGTGCTGG | 24 |
| 5 | OPA-05 | AGGGGTCTTG | 15 | 15 | OPA-15 | TTCCGAACCC | 25 |
| 6 | OPA-06 | GGTCCCTGAC | 16 | 16 | OPA-16 | AGCCAGCGAA | 26 |
| 7 | OPA-07 | GAAACGGGTG | 17 | 17 | OPA-17 | GACCGCTTGT | 27 |
| 8 | OPA-08 | GTGACGTAGG | 18 | 18 | OPA-18 | AGGTGACCGT | 28 |
| 9 | OPA-09 | GGGTAACGCC | 19 | 19 | OPA-19 | CAAACGTCGG | 29 |
| 10 | OPA-10 | GTGATCGCAG | 20 | 20 | OPA-20 | GTTGCGATCC | 30 |

To avoid embryonic contamination, the embryos were removed under the light microscope from the implantation sites. After the DDPCR analysis, the differential display pattern was further verified by the Northern blotting analysis and cDNAs from those confirmed bands were sub-cloned into the pGEM-T vector (Promega, Madison, WI, USA) and sequenced manually.

For Northern blot analysis, no attempt was made to separate the embryos from the decidua before day 8 of pregnancy, but for 8- and 11-day pregnant mice, embryos were separated from the uterine tissue. Total RNA was extracted from whole uteri or from pools of implantation or inter-implantation sites by the acid guanidinium thiocyanate-phenol-chloroform extraction (GTC) method (Chomczynski & Sacchi, 1987). RNA (10-15 (g) was denatured at 50°C for 60 min in 50% dimethylsulfoxide (DMSO) and 1 M glyoxal, and the denatured RNA was fractionated by electrophoresis through a 1.2% agarose gel in 10 mM sodium phosphate buffer (pH 7.0) and transferred to positively charged nylon membranes (Hybond-N+, Amersham) by overnight capillary blotting in 5 x SSPE (1 x SSPE = 150 mM NaCl, 10 mM NaH2PO4, 1 mM EDTA, pH 7.4). Membranes were baked at 80°C for 2 h followed by 3 min UV cross linking.

Transcript size was estimated by comparison with RNA size standards (Gibco-BRL, Gaithersburg, MD USA). A simplified filter paper sandwich blotting method (Jones & Jones, 1992; Nie et al, 2000b) was used for the hybridisation process at 42°C overnight, without a prehybridisation step. The hybridisation buffer contained 50% formamide, 6X SSPE (900 mM sodium chloride, 60 mM sodium phosphate, pH 7.4, 6 mM EDTA), 5X Denhardt's solution (0.1% Ficoll, 0.1% polyvinylpyrrolidone, 0.1% bovine serum albumin), 0.5% SDS, 0.1 mg/ml sheared herring sperm DNA, and ³²P-dCTP-labelled probes. The radio-labeled cDNA probes were generated by random primer labeling of 25 ng cDNA with [³²P]deoxy-CTP (50 (Ci/reaction).

Unincorporated nucleotides were removed with a MicroSpin S-200 HR column (AMRAD Pharmacia Biotech, Melbourne, Australia). Following hybridisation, the blots were rinsed twice with 5 x SSPE at 37°C, then twice for 15 min each at 37°C with 2 x SSC/0.1 % SDS (w/v) (1x SSC = 150 mM NaCl, 15 mM Na₃ citrate, pH 7.4). In some cases, additional washes were also performed with 0.5 or 1 x SSC/0.1 % SDS for 15 min at 60°C. To determine lane to lane loading variation, each blot was also probed with a mouse cDNA probe for glyceraldehyde-3-phosphate dehydrogenase (GAPDH) or 18S ribosomal RNA. Between hybridisations, blots were stripped by incubation at 80°C for 3 h in 1 mM EDTA /0.1 % SDS followed by rinsing in H₂O.

For reverse transcriptase-polymerase chain reaction (RT-PCR), 1 µg DNA-free total RNA was reverse-transcribed at 46°C for 1-1.5 h in 20 µl reaction mixture, using 100 ng random hexanucleotide primers and AMV reverse transcriptase (Boehringer-Mannheim, Nunawading, Vic., Australia) with the cDNA synthesis buffer. The PCR was performed in a total volume of 40 µl with 1-1.5 µlof the RT reaction, 1x PCR buffer, 20 µMdNTPs, 10 pmol forward and reverse primers and 2.5 units of Taq DNA polymerase (Boehringer-Mannheim), in 3 stages as follows:
(a) one cycle of an incubation for 5 min at 95°C, 1 min at 52°C-60°C, and 2 min at 72°C;
(b) 32 cycles with a denaturation for 45 sec at 95°C, annealing at 52°C-60°C for 50 sec and extension at 72°C for 1 min; and
(c) incubation for 5 min at 72°C.

The PCR products were subjected to electrophoresis (100v/0.5 x TBE buffer)on 1.5% agarose gel, and stained with ethidium bromide. Bands of interest were cut out from the agarose gels, purified with the Qiaquick gel extraction kit (Qiagen Pty Ltd., Clifton Hill, Vic., Australia), cloned into a pGEM-T easy vector (Promega) according to the manufacturer's instructions and sequenced on an automated sequencer (Applied Biosystems, ABI Prism™, 377 DNA Sequencer) using the ABI Prism BigDye terminator cycle sequencing ready reaction kit.

The primer sequences used to amplify the following fragments of mouse proprotein convertase PC6 (MUSPC6 GenBank Accession No.:D12619; 2994 bp)) from mouse uterine RNA were:

| | | |
|---|---|---|
| (1) MUSPC6-fragA (171 bp), upper primer | | |
| | 5' GTT AGT TGT GCG CGC TGC TTA 3' [nucleotide (nt) 4-24] | SEQ ID NO: 31 |
| and lower primer | | |
| | 5' GCG CGT CCG GCA TAC C 3' (nt 159-174) | SEQ ID NO: 32 |
| | (2) MUSPC6-fragB (422 bp), upper primer 5' CAC CCA TCC TTG CCA GTC AG 3' (nt 501-520) | SEQ ID NO: 33 |
| and lower primer | | |
| | 5' CAT CCA CAG TCT TGC CAT CGT 3' (nt 902-922). | SEQ ID NO: 34 |
| | (3) MUSPC6-fragD (656 bp), upper primer 5' GAT CGG TGC ACT GAA GGA CTA 3' (nt 267-287) | SEQ ID NO: 35 |
| and lower primer | | |
| | 5' CAT CCA CAG TCT TGC CAT CGT 3' (nt 902-922); | |
| this fragment was digested with HindII, and two smaller fragments (177 bp and 479 bp) were obtained; the 177 bp was then designated as MUSPC6-fragDA and the 479 bp as MUSPC6-fragDB. | | |
| | (4) MUSPC6-fragG(653 bp), upper primer | |
| | 5' CTG GCA GCA TGT TAT TGT CA 3' (1305-1324) | SEQ ID NO: 36 |
| and lower primer | | |
| | 5' CGT AGT CAT CTG TGG GGT CTT 3' (1937-1957). | SEQ ID NO: 37 |
| | (5) MUSPC6-fragH (672 bp), upper primer | |
| | 5' CCA CTA CCA TGC TGA CAA GA 3' (nt 2106-2125) | SEQ ID NO: 38 |
| and lower primer | | |
| | 5' AGA ACT TTT CGC TGA CAG AGA 3' (nt 2757-2777) | SEQ ID NO: 39 |
| | (6) MUSPC6-fragl (227 bp), upper primer | |
| | 5' GTA TGC TTG TGA AAA AGA ACA 3' (nt 2735-2755) | SEQ ID NO: 40 |
| and lower primer | | |
| | 5' CCC TGC ACA AAC TTG AGA TAG 3' (nt 2941-2961) | SEQ ID NO: 41 |
| The following two fragments were cloned by RT-PCR from mouse intestine RNA on the basis of the MUSPC6B cDNA sequence (Accession: D17583, 5208 bp): | | |
| | (1) MUSPC6-fragK (377 bp), upper primer | |
| | 5' TGG AAG GCA AGG ACT GGA ATG 3' (nt 2522-2542) | SEQ ID NO: 42 |
| and lower primer | | |
| | 5' CTC TGG GCA ACT TGT GTA GCA 3' (nt 2878-2898) | SEQ ID NO: 43 |
| | (2) MUSPC6-fragM (398 bp), upper primer | |
| | 5' AGG GAG GCT GAG TTT TAC GAG 3' (nt 4285-4305) | SEQ ID NO: 44 |
| and lower primer | | |
| | 5' GTG GGT GGT GGG TTT GAG 3' (nt 4665-4682). | SEQ ID NO: 45 |

### Example 2: Analysis of MUSPC6 Gene by Southern Blotting

Total genomic DNA was isolated from non-pregnant uterus and from kidney, using the DNeasy Tissue Kit (Qiagen Pty Ltd). A total amount of 10 µgwas then digested separately with an excess of several restriction endonucleases (BamH1, EcoR1, HindIII, and BgIII) at 37°C for 14 hours, and fractionated on 0.8% agarose gel. The DNA was then blotted on to positively charged nylon membranes (Hybond-N, Amersham) using the standard Southern blotting procedure and probed with radio-labeled cDNA, as described for the Northern analysis.

### Example 3: Artificial decidualization of mouse uterus and in situ Hybridisation

To induce artificial decidualization in the mouse uterus, non-pregnant mice were ovariectomised and treated with estradiol and progesterone as described by Finn and Pope (Finn and Pope. 1994), and uteri collected 48 hours after the administration of oil stimulus. Sense and anti-sense digoxygenin (DIG)-labelled RNA probes against PC 5/6 (clone 9.5) were generated using the DIG RNA Labeling kit (Boehringer Mannheim), and the concentrations determined according to the manufacturer's instructions. Five micron sections of formalin-fixed paraffin-embedded tissues were subjected to in situ hybridisation as described by (Komminoth, 1992). All sections were counterstained with Mayer's hematoxylin.

### Example 4: DDPCR Analysis and Confirmation of Clone 9.5 by Northern Blotting

To identify genes which are potentially critical for the initial process of embryo implantation in the mouse, we compared the gene expression pattern of implantation and interimplantation sites in the uterus on day 4.5 of pregnancy, using the DDPCR technique. Seventeen bands, of which the intensities were different between the two sites, were detected on DDPCR gels (Nie et al., 2000). One of these bands, band 9, was fully analysed.

On the DDPCR gel, band 9 was much more intense in implantation sites compared to interimplantation sites in all individual animals tested as shown in Figure 1A. To verify that this band indeed represents gene(s) which are differentially expressed between the two sites, the cDNA products of band 9 were extracted out of the DDPCR gel, re-amplified and cloned into the pGEM-T vector; Northern blot analysis was performed using the cloned inserts as probes. Among the clones analysed, cDNA of clone 9.5 explicitly detected differential expression of mRNA between the two sites on the Northern blot, with much higher mRNA level present in implantation sites compared to interimplantation sites. This is illustrated in Figure 1B. On this initial Northern blot, more than one transcript were observed, with a 3.5 kb band being the prominent transcript. This confirmed that clone 9.5 contained the cDNA representing the original expression pattern of band 9 on the DDPCR gel.

### Example 5: Sequence Analysis of Clone 9.5 and comparison with the Genbank Database

Band 9 resulted from the DDPCR amplification of day 4.5 implantation site mRNA with the following two primers: 5' primer, GTGACGTAGG (OPA-8, Table 1) and 3' primer, T12MA (Table 1). After the confirmation that clone 9.5 contained the cDNA representing band 9, the nucleotide sequence of this clone was determined, and is shown in Table 2. It contained 158 nucleotides, and the ends of the sequence indeed contained the unique and expected primer sequence of GTGACGTAGG at the 5' end, and the reverse complementary sequence of T12MA at the 3' end (underlined in Table 2). This confirmed that the cDNA in clone 9.5 was indeed the direct PCR product amplified from the specific primers applied during DDPCR amplification.

**Table 2.**

| |
|---|
| The sequence of clone 9.5 (158 bp) derived from band 9 of DDPCR analysis |
| 1 GTGACGTAGG ACAGATCGGT GCACTGAAGG ACTACTATCA CTTCTACCAT 51 AGTAGGACCA TTAAAAGGTC TGTTCTCTCG AGCAGAGGAA CCCACAGTTT 101 CATTTCAATG GAACCAAAGG TGGAGTGGAT CCAACAGCAA GTGGTGAAAA 151 AAAAAAAA SEQ ID NO: 46 |

| |
|---|
| * The underlined parts represent the primers used during DDPCR amplification |

When compared to the GenBank database, the sequence of clone 9.5 showed 98% identity to mouse serine protease PC6 (MUSPC6)(Accession: D12619; 2994 bp), 98% identity to mouse convertase PC5 (MUSPC5)(Accession: L14932; 2848 bp) and 98% identity to human protease PC6 isoform A (HPC6A)(Accession: U56387; 2844 bp). It appears that in fact MUSPC5 and MUSPC6 are actually the same gene under different names, and that they are 90% homologous to HPC6A cDNA. The detailed comparison of clone 9.5 (SEQ ID NOS: 47 and 48) with MUSPC6 cDNA (SEQ ID NOS: 49 and 50) is illustrated in Table 3, showing that clone 9.5 aligned to nucleotide (nt) 254-411 of MUSPC6 cDNA. In principle, and in most experimentally-tested cases, a DDPCR-derived fragment should represent the 3' end sequence for a cDNA (Liang & Pardee, 1992; Nie et al. 2000); however, in contrast to this expectation, clone 9.5 aligns with the nucleotide sequence 254-411, which is near the 5' end, but not to the 3' end of MUSPC6 cDNA (Table 3). This unusual outcome is thought to result from the unique sequence of MUSPC6 cDNA (Table 3) and the intrinsic nature of PCR, in which the 5' end of a primer sequence can be degenerate.

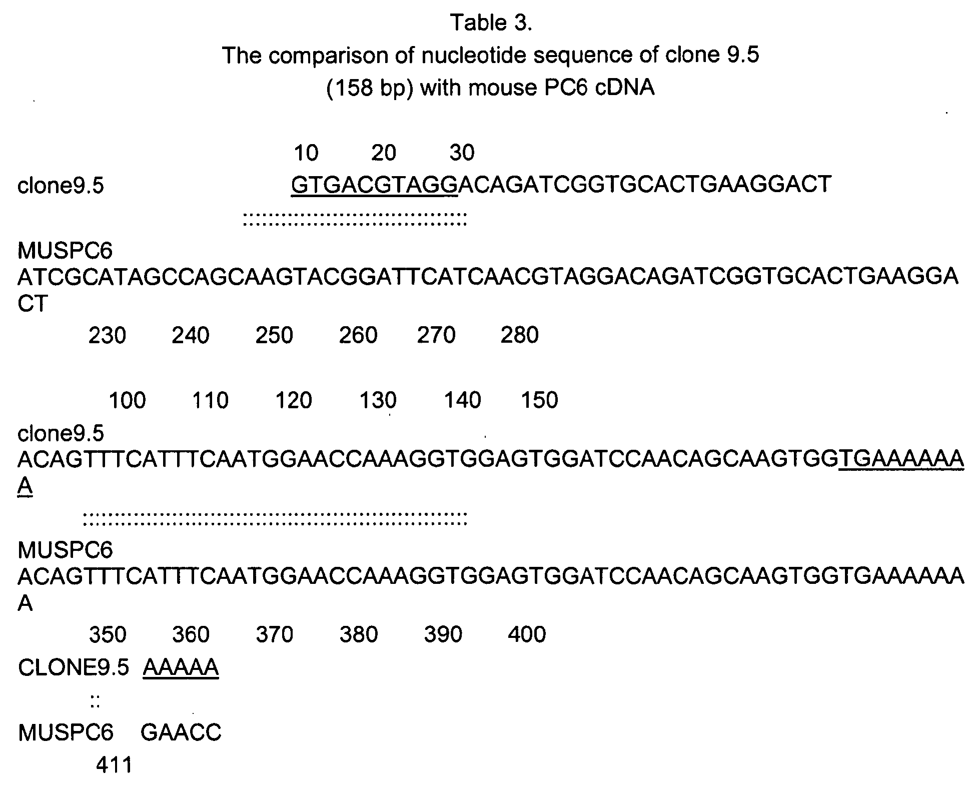

### Example 6: RT-PCR Cloning of Mouse PC6 (MUSPC6) and Determination of MUSPC6 mRNA Expression in the Uterus During Early Pregnancy

The results shown in Figure 1B and Table 3 indicated that clone 9.5 represents part of MUSPC6 cDNA, and that the level of expression of this gene is much higher in implantation sites compared to the interimplantation sites on day 4.5 of pregnancy. To confirm the identity of clone 9.5 and verify that MUSPC6 mRNA is indeed differentially expressed between the two sites, two additional cDNA fragments of MUSPC6, designated MUSPC6-fragA and MUSPC6-fragB, were cloned using the RT-PCR approach, and used as cDNA probes on the Northern blots. MUSPC6-fragA was a 171 bp fragment covering nucleotides 4-174, and MUSPC6-fragB was a 422 bp fragment covering nucleotides 501-922 of MUSPC6 cDNA (Accession: D12619; 2994 bp). These two fragments were designed to represent the up-and down-stream region of MUSPC6 cDNA, to which clone 9.5 is aligned. Using MUSPC6-fragA and MUSPC6-fragB as probes, a pattern of mRNA expression similar to that shown in Figure 1B, where clone 9.5 was used as a probe, was observed between implantation sites and interimplantation sites (data not shown). This conclusively confirmed the identity of clone 9.5 as representing MUSPC6, and showed that MUSPC6 mRNA is upregulated in implantation sites on day 4.5 of pregnancy.

In a subsequent experiment, MUSPC6-fragB was used as the cDNA probe, and additional Northern analyses were performed to determine the expression pattern of this gene in the uterus in relation to the time of implantation and early pregnancy. Total RNA from the uterus of non-pregnant mice (estrus) and pregnant mice at the initial stage for implantation (day 4.5 of pregnancy) through to fully established implantation and placentation (day 10.5 of pregnancy) was analysed. The results are shown in Figure 2. Very low expression was observed in non-pregnant mice, as well as in mice on day 3.5 of pregnancy. However, around day 4.5 up to day 6.5 of pregnancy a dramatic upregulation of this gene occurred in the implantation sites, but not in the interimplantation sites (Figure 2). Beyond day 6.5 of pregnancy, the expression level then drastically decreased, and returned to the level in non-pregnant uterus.

Thus this upregulation was very transient, and only occurred between day 4.5 and 6.5 of pregnancy, when the embryos are actively implanting into the uterus. The upregulation was very implantation site-specific, and did not occur uniformly along the uterine horns, but only in the implantation sites.

In addition, it was noticeable that more bands were observed in Figure 2 than in Figure 1 B. This was simply due to the different lengths of film exposure for the two Northern blots. After the experiment reported in Figure 2, the result of Figure 1B was repeated, and similar numbers of bands were detected after much longer film exposure. Thus it is clear that in implantation sites during the active embryo implantation period, the uterus expresses multiple transcripts of MUSPC6, with a size range of 2.2 kb, 3.5 kb, 6.5 kb and 10 kb; of these the 3.5 kb transcript is the most abundant. This observation agrees with the finding that multiple transcripts were detected for MUSPC6 in the brain and intestine (Nakagawa et al. 1993 a & b).

### Example 7: MUSPC6 mRNA Level during the Estrous Cycle

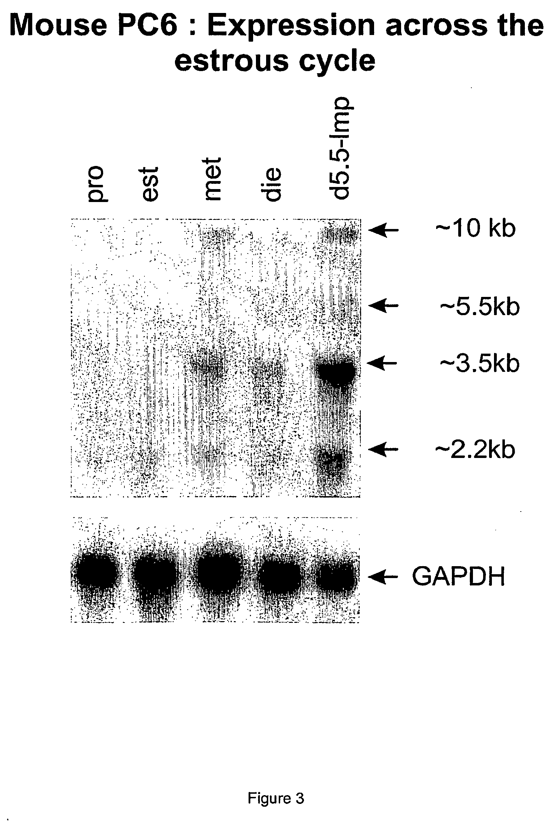

The influence of the estrous cycle on the expression of MUSPC6 in the non-pregnant uterus was examined by determining the level of MUSPC6 mRNA by Northern analysis. The study utilised 16 individual mice at different stages of the cycle (metestrus, diestrus, proestrus and estrus), grouped to represent 4 cycles, and results from one typical cycle are shown in Figure 3. In all cases, the expression level was very low at estrus and proestrus and became marginally higher during metestrus and diestrus; however, the levels at all stages of the estrous cycle were much lower than that in the implantation sites during the implantation period. These results indicated that the upregulation observed in implantation sites is not solely a direct result of the influence of the ovarian hormones during pregnancy, but requires other factors, such as the presence of an embryo.

### Example 8: Effects of the Embryo on Uterine Expression of MUSPC6

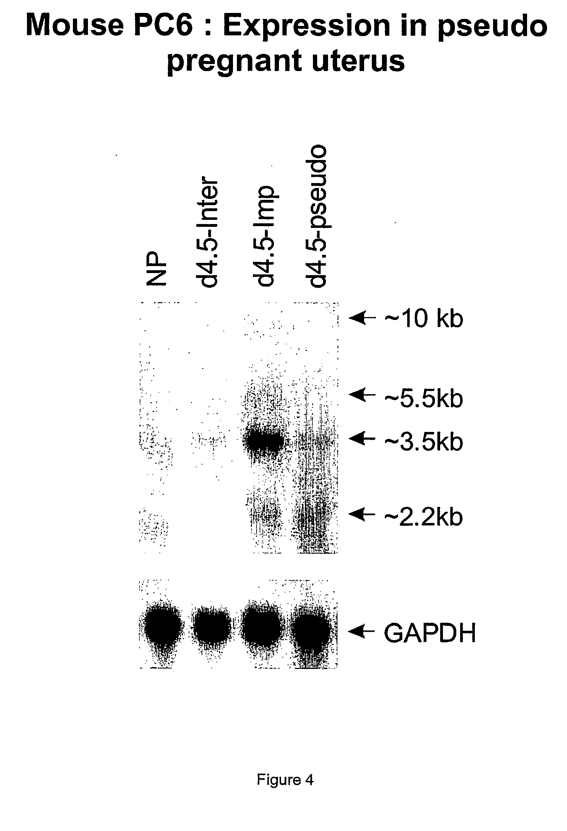

To determine whether the presence of an embryo in the uterus was essential for the observed changes in MUSPC6 mRNA expression during early pregnancy, total RNA was isolated from mice on day 4.5 of pseudopregnancy, and the mRNA expression pattern compared with that in normal day 4.5 pregnant animals by Northern analysis. The results are shown in Fig 4. This experiment demonstrated that the mRNA level in the pseudopregnant mice was actually equivalent to that in interimplantation sites of pregnant animals, and was much lower than that at the implantation sites. This implied that the observed increase in MUSPC6 mRNA expression at the implantation sites during early pregnancy required the presence of embryos in the uterus.

### Example 9: Tissue Distribution of MUSPC6 mRNA

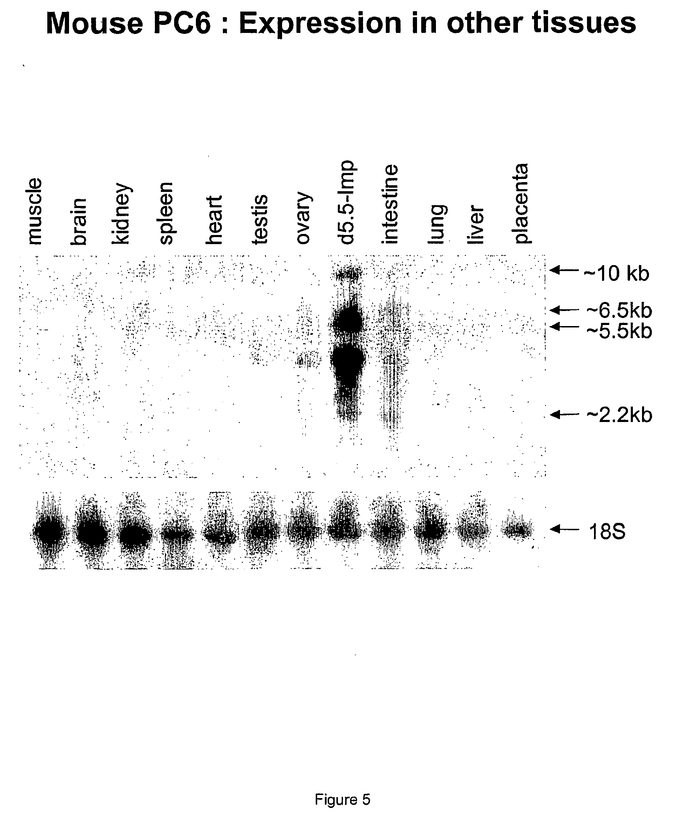

Multi-tissue Northern analysis was performed to investigate the tissue distribution of MUSPC6 mRNA expression. The results, illustrated in Figure 5, show that MUSPC6 is not widely expressed. When an equal amount of total RNA was compared, the implantation sites on day 5.5 showed the highest level of expression, and apart from the uterus, only intestine, ovary, testis and brain among the 12 tissues tested showed detectable expression.

### Example 10: Evidence from a Uterine- and Pregnancy-Specific form of MUSPC6

MUSPC6 has been previously studied in the brain and intestine; therefore intestine was chosen as the positive control tissue for our studies. However, it was observed that the profile of the mRNA transcripts detected by Northern blotting was different in the uterus from that in the intestine. Both tissues show multiple transcripts ranging from 2.2 kb to 10 kb, but there is a subtle difference; the 6.5 kb transcript present in the intestine is replaced by a 5.5 kb one in the uterus, as shown in Figure 2. This 6.5 kb intestinal transcript has been previously investigated in detail, and shown to encode a MUSPC6 isoform, designated as mouse PC6 B (Accession: D17583, 5208 bp), which has an extremely large cysteine-rich motif. This isoform of MUSPC6 (MUSPC6B) is membrane-bound, whereas the previously documented MUSPC6 is soluble. Consequently, in order to avoid confusion, the previously documented isoform of MUSPC6 disclosed herein is designated MUSPC6A, and the membrane-bound isoform is designated MUSPC6B. From comparison of the cDNA and protein sequences, it appears that MUSPC6A and MUSPC6B are generated via alternative splicing of the same primary transcript. It appears that the 5.5kb mRNA transcript which we have identified in the uterus is unique to the pregnant uterus, and may encode an isoform of PC6 which is different from PC6A or PC6B.

In order to confirm the presence of a unique 5.5 kb transcript in the implantation sites in the uterus, and to determine whether this 5.5 kb transcript is different from the 6.5 kb transcript present in the intestine and therefore may code for another isoform of MUSPC6, the following approaches were used.

Firstly, cDNA fragments coding for the different domains of mouse MUSPC6 protein were RT-PCR cloned, and these fragments were used as cDNA probes for Northern blotting performed on mouse uterine tissues and the intestine. At the protein level, the MUSPC6 protein consists of the following five domains (in the order from the N- to C-terminal): signal peptide, propeptide, subtilisin-like catalytic domain, homo B domain and cysteine (Cys)-rich domain. The first four domains are exactly the same between the MUSPC6A and MUSPC6B isoforms, and differences are found only in the Cys-rich domain, which is at the C-terminus. There are two unique features of the B isoform:
(1) It has a very long Cys-rich domain, which is about four times large as that of the A isoform,
(2) It contains a stretch of hydrophobic amino acids as a putative trans-membrane domain near the C-terminus (Nakagawa et al. 1993).

Based on this protein domain information, the following cDNA fragments were cloned using RT-PCR:
(1) MUSPC6-fragA, 171 bp, covering nt 4-174 of MUSPC6A cDNA (Accession: D12619; 2994 bp), representing the 5'- untranslated region (UTR) and the signal peptide domain;
(2) MUSPC6-fragDA, 177 bp, covering nt 267-443 of MUSPC6A cDNA, representing the propeptide domain;
(3) MUSPC6-fragDB, 479 bp, covering nt 444-922 of MUSPC6A cDNA, representing the subtilisin-like catalytic domain;
(4) MUSPC6-fragG, 653 bp, covering nt 1305-1957 of MUSPC6A cDNA, representing the Homo-B domain;
(5) MUSPC6-fragH, 672 bp, covering nt 2106-2777 of MUSPC6A cDNA, representing the common Cys-rich domain present in both the A and B isoforms;
(6) MUSPC6-fragl, 227 bp, covering nt 2735-2961 of MUSPC6A cDNA, representing the C-terminus and 3' UTR of the A isoform, and thus representing a cDNA fragment specific to the A isoform only;
(7) MUSPC6-fragK, 377 bp, covering nt 2522-2898 of MUSPC6B cDNA (Accession: D17583, 5208 bp), representing the unique Cys-rich domain of the B isoform;
(8) MUSPC6-fragM, 398 bp, covering nt 4285-4682 of MUSPC6B cDNA, representing the unique trans-membrane domain of the B isoform.

Northern blots containing mouse uterine tissues of non-pregnant (estrus), implantation and interimplantation sites on day 4.5 and 5.5 of pregnant mice and intestine were prepared and probed with the cDNA fragments described above. The results are shown in Figure 6. When the fragments MUSPC6-fragA/DA/DB/G/H were used as probes, all of the multiple bands observed previously for the uterus and intestine were detected in both type of tissues, and the 6.5 kb B-isoform specific transcript was detected only in the intestine, as shown in Figure 6A. In all cases, a 5.5 kb uterus-specific transcript was indeed present in the implantation sites, and this transcript seems to have replaced the 6.5 kb transcript present in the intestine, as shown in Figure 6A.

This indicates that the cDNA sequences tested so far are common to all of the multiple transcripts, and that therefore the protein domains represented by these cDNAs are common to all of the possible isoforms of the MUSPC6 protein. These results also confirmed that the mRNA transcript profiles are different between the uterus and the intestine. When MUSPC6-fragl, an A-isoform specific cDNA fragment, was used as a probe on the same blot, only the 3.5 kb transcript, but not the other transcripts, was detected in all of the tissues; certainly this probe did not detect the 6.5 kb transcript of the intestine, as shown in Figure 6B, indicating that only the 3.5 kb transcript but not the other ones contains this A-isoform specific cDNA sequence. This result also confirms the known difference between the A and B isoforms at the cDNA level. When the two B-isoform specific cDNA fragments, MUSPC6-fragK and MUSPC6-fragM, were used on the same blot, in both cases the 6.5 kb transcript was detected only in the intestine; no other bands were observed in either the intestine or the uterus, as shown in Figure 6C.

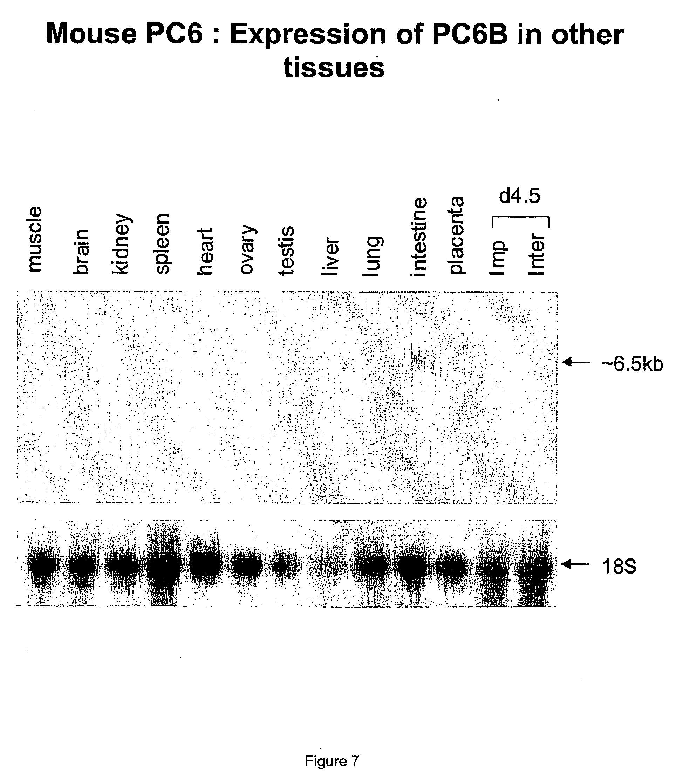

When these B-isoform specific probes were tested on the multi-organ Northern blot, only the intestine showed a clear 6.5 kb band; other tissues, including muscle, brain and uterus were negative. These results are shown in Figure 7, and both confirmed the specificity of the probes used in this study, and verified that the B-isoform is only expressed in the intestine, and not in the uterus or other organs.

Collectively, our results confirm that:
(a) the 5.5 kb transcript detected in the uterus is different from the 6.5 kb one in the intestine,
(b) this 5.5 kb transcript is indeed specific to the implantation sites of the uterus during the embryo implantation period, and
(c) this uterine-specific transcript may code for an isoform of MUSPC6 which is different from the previously identified A and from the B isoforms, the difference is mainly at the C-terminal end.

### Example 11: Genomic Southern Analysis of MUSPC6 Gene

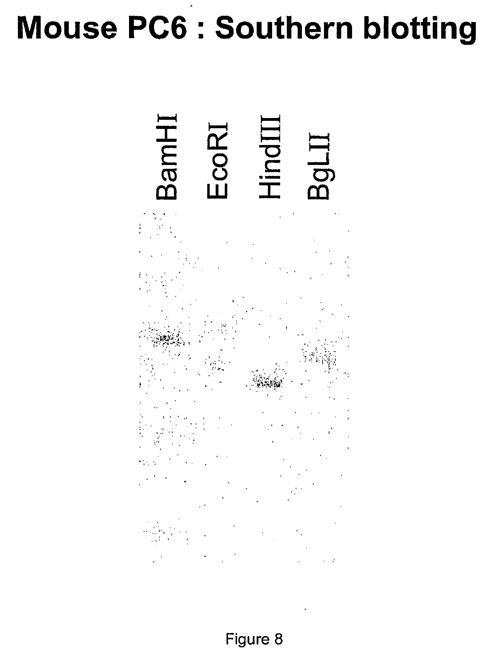

Genomic DNA was isolated from mouse uterus and kidney, and the DNA was subjected to Southern analysis. Similar results were obtained for the two tissue types; thus only the results obtained with the uterus will be discussed. Figure 8 shows the results of Southern analysis of mouse genomic DNA digested separately with BamHI, EcoRI, HindIII and Bg1II and probed with radio-labeled MUSPC6-fragH. In all cases, the digestion pattern was quite simple, and indicate that the MUSPC6 gene is represented by a single copy in the genome.

### Example 12: In situ Hybridisation of MUSPC6 mRNA in the Uterus of Mice during Early Pregnant

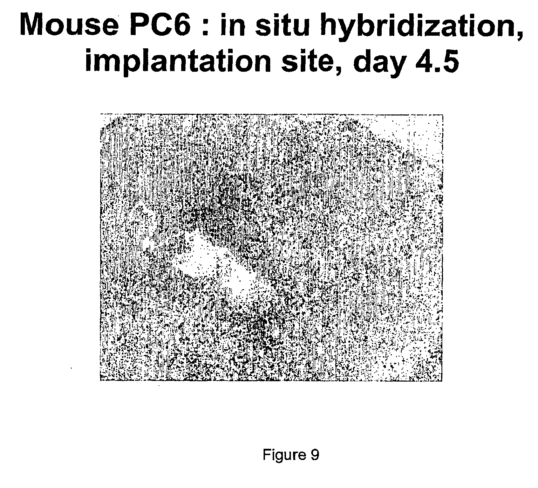

The cell types which express MUSPC6 mRNA in the uterus were identified by in situ hybridisation, using riboprobes generated against clone 9.5. The sequences of the riboprobes are set out in Table 2. In non-pregnant and in day 3.5 pregnant uterus, no single cell type was distinctively positive. Positive signals were only detected from day 4.5-6.5 of pregnancy at the implantation sites, and they were predominantly localised in the decidualized stromal cells at the anti-mesometrial pole of the uterus, as shown in Figure 9; throughout this period of time, the interimplantation sites were always negative. Interestingly, at the implantation sites the mesometrial side of the uterus on the same section always had many fewer positive cells, compared to the anti-mesometrial pole. The intensity of the signals was high on day 4.5 and 5.5, and then decreased dramatically on day 6.5. After day 6.5 of pregnancy, no signals were detected in any cells in the uterus.

These results indicate that MUSPC6 mRNA is only expressed in the decidual zones of the uterus around the embryo implantation period, and that this expression is quite transient, and specific to the decidua. The expression is not uniformly distributed at the implantation sites; the anti-mesometrial pole, where the embryo contacts with the uterus during implantation, showed higher expression, and the opposite side, the mesometrial pole, showed a much lower level of expression. Interestingly, some of the cells in artificially decidualized uterus were also positive for MUSPC6 probe. This indicates that MUSPC6 is involved in the decidualization process, and that it may not be restricted to the embryo-induced decidualization, but also occurs in other decidualization events which can be induced by other means, such as oil.

### Example 13: Detection of PC6 in human uterus and other human tissues

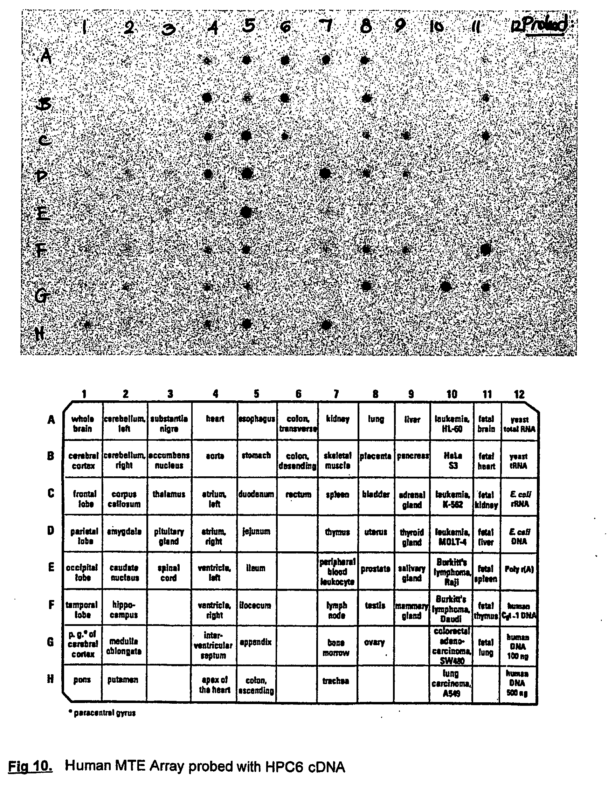

A human PC6 cDNA probe was prepared by RT-PCR and cloned, and applied to a human multi-tissue array (Clontech). The results are illustrated in Figure 10. A strong positive signal was detected in the heart, gastrointestinal tract, kidney, thymus, lung, placenta, uterus, testis, ovary, in similar fetal organs, and in colorectal adenocarcinoma.

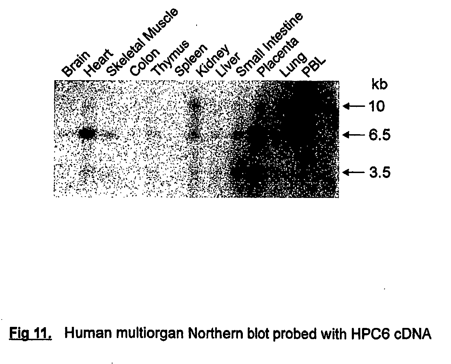

A human multiorgan Northern blot with 1(g of polyA+ RNA (Clontech), applied to each lane (was probed as shown in Figure 11. Strong positive signals were obtained for heart, kidney, small intestine, placenta, and lung. Transcript sizes detected were around 10kb, 6.5kb and 3.5 kb.

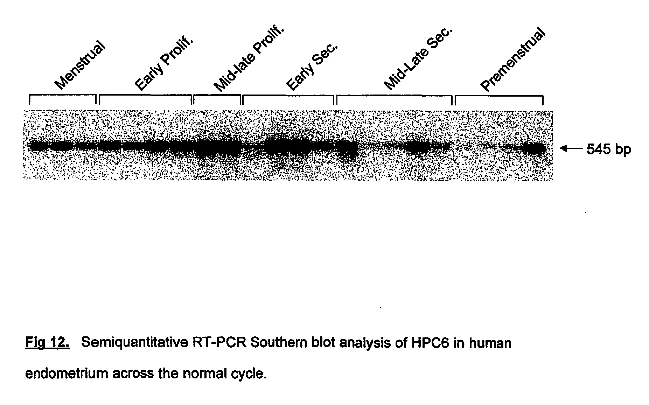

Figure 12 shows the results of semi-quantitative RT-PCR Southern blot analysis of PC6 in human endometrium taken across the menstrual cycle. Primers used were upper primer: 5' GAT CGG TGC ACT GAA GGA CTA 3' (SEQ ID NO: 35), lower primer: 5' CCA GCA TTC GCA CTC CTC 3' (SEQ ID NO: 51). An expected band of 545bp was detected in all samples.

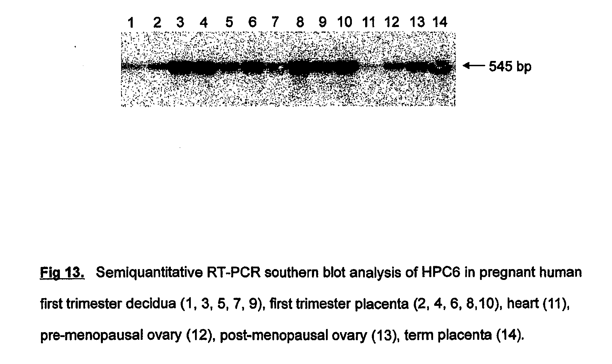

Figure 13 shows the results of similar semiquantitative RT-PCR analysis in first trimester decidua and placenta, heart, pre- and post-menopausal ovary and term placenta. Thus it is apparent that PC6 is expressed in the human endometrium and a number of other human tissues. Its expression in decidua and placenta of early pregnancy suggests a role in human pregnancy.

### Example 14: Expression of PC6 in human endometrial cancers.

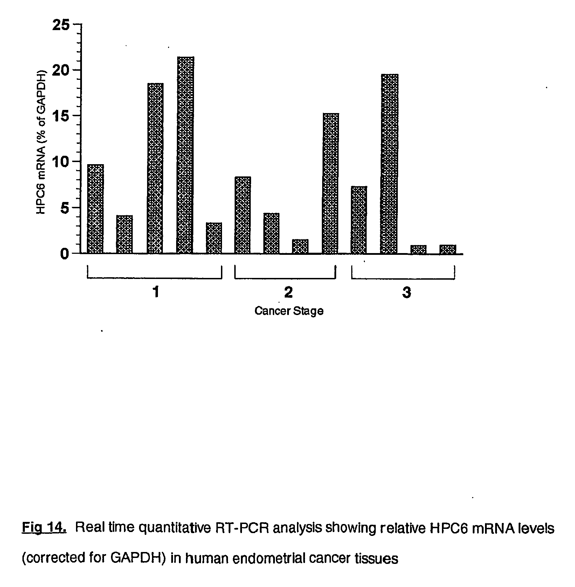

Figure 14 shows the results of real-time quantitative RT-PCR performed using primers on 13 samples of endometrial cancer. Messenger RNA values for PC 5/6 have been corrected for loading, as assessed by quantitation for glyceraldehyde phosphate dehydrogenase. PC6 was detected in every cancer sample, and for each grade of the cancer (1-3), International Federation of Gynaecology and Obstetrics Classification).

It will be apparent to the person skilled in the art that while the invention has been described in some detail for the purposes of clarity and understanding, various modifications and alterations to the embodiments and methods described herein may be made without departing from the scope of the inventive concept disclosed in this specification. References cited herein are listed below:
Boudreault A et al. Proprotein convertase PC1/3-related peptides are potent slow tight-binding inhibitors of murine PC1/3 and Hfurin. J Biol Chem. 1998 Nov 20;273(47):31574-80.
Chomczynski P and Sacchi N Single-step method of RNA isolation by acid guanidinium thiocyanate-phenol-chloroform extraction. Anal.Biochem. 1987; 162: 156-159.
Finn, C.A. and Pope, M. Vascular and Cellular changes in the decidualized endometrium of the ovariectomized mouse following cessation of hormone treatment: a possible model for menstruation. J. Endrocrinol. 1984; 100: 295-300.
Jean F et al. Alpha1-Antitrypsin Portland, a bioengineered serpin highly selective for furin: application as an antipathogenic agent. Proc Natl Acad Sci U S A. 1998 Jun 23;95(13):7293-8.
Komminoth P Digoxigenin as an alternative probe labeling for in situ hybridization. Diagn Mol Pathol1992; 1: 142-150.
Liang P and Pardee AB Differential display of eukaryotic messenger RNA by means of the polymerase chain reaction. Science 1992; 257: 967-970.
Liang P and Pardee AB Distribution and cloning of eukaryotic mRNAs by means of differential display: refinement and optimization. Nucleic Acids Res.1993; 14: 3269-3275.
Nie G-Y et al. Hormonal and non-hormonal agents at implantation as targets for contraception. Reprod Fertil Dev 1997; 9: 65-76.
Nie G-Y et al. Complex regulation of calcium-binding protein D9k (Calbindin-D9k) in the mouse uterus during early pregnancy and at the site of embryo implantation. Biol Reprod. 2000a; 62 : 27-36.
Nie G-Y et al. Identification of monoclonal nonspecific suppressor factor beta (MNSFbeta) as one of the genes differentially expressed at implantation sites compared to interimplantation sites in the mouse uterus. Mol Reprod Dev. 2000b; 55: 351-363.
Zhong M et al. The prosegments of furin and PC7 as potent inhibitors of proprotein convertases. J Biol Chem. 1999; 274:33913-33920.

### PREFERRED EMBODIMENTS

Particularly preferred embodiments of the invention are given below:
19. A nucleic acid molecule encoding an isoform of proprotein convertase 5/6 which:
   (a) is present at the implantation sites in pregnant uterus during the implantation period but not in intestine or other tissues known to contain PC 5/6;
   (b) encodes an RNA transcript of about 5.5 kb; and
   (c) is able to hybridize under at least moderately stringent conditions to the sequence: 
1 GAAGTTAGTT GTGCGCGCTG CTTAGCGCGC GAGCCAGCGG GCGGGCGAAG
51 GCGGCGAAGC GTCGGGACCA TGGACTGGGA CTGGGGGAAC CGCTGCAGCC
101 GCCCGGGACG GCGGGACCTG CTGTGCGTGC TGGCACTGCT CGCCGGCTGT
151 CTGCTCCCGG TATGCCGGAC GCGCGTCTAC ACCAACCACT GGGCAGTGAA
201 GATCGCCGGC GGCTTCGCGG AGGCAGATCG CATAGCCAGC AAGTACGGAT
251 TCATCAACGT AGGACAGATC GGTGCACTGA AGGACTACTA TCACTTCTAC
301 CATAGTAGGA CCATTAAAAG GTCTGTTCTC TCGAGCAGAG GAACCCACAG
351 TTTCATTTCA ATGGAACCAA AGGTGGAGTG GATCCAACAG CAAGTGGTGA
401 AAAAAAGAAC CAAGAGGGAT TATGACCTCA GCCATGCCCA GTCAACCTAC
451 TTCAATGATC CCAAGTGGCC AAGTATGTGG TACATGCACT GCAGTGACAA
501 CACCCATCCT TGCCAGTCAG ACATGAATAT CGAAGGAGCC TGGAAGAGAG
551 GCTACACGGG GAAGAATATC GTGGTGACTA TCCTGGATGA CGGCATCGAG
601 AGAACCCACC CAGATCTGAT GCAAAACTAC GATGCTCTGG CAAGTTGCGA
651 TGTGAATGGG AATGACTTGG ACCCGATGCC TCGTTATGAT GCAAGCAATG
701 AGAACAAGCA TGGGACCCGC TGTGCCGGAG AAGTGGCAGC CACTGCAAAC
751 AACTCTCACT GCACTGTCGG GATCGCTTTC AACGCCAAGA TTGGAGGTGT
801 GAGAATGCTG GATGGTGATG TCACTGACAT GGTGGAGGCA AAGTCTGTCA
851 GCTACAACCC ACAGCATGTG CACATCTACA GTGCCAGCTG GGGACCAGAT
901 GACGATGGCA AGACTGTGGA TGGGCCAGCT CCCCTCACCC GGCAAGCCTT
951 TGAGAATGGC GTGAGAATGG GGCGGAGAGG CCTTGGATCT GTGTTTGTGT
1001 GGGCATCTGG CAATGGTGGA CGGAGCAAGG ATCACTGTTC TTGTGATGGC
1051 TATACCAACA GCATCTATAC CATCTCCATC AGCAGTACGG CCGAAAGTGG
1101 AAAGAAACCT TGGTACTTGG AAGAGTGTTC ATCTACACTG GCTACAACCT
1151 ACAGCAGTGG AGAATCCTAT GATAAGAAAA TAATCACTAC TGATCTAAGG
1201 CAGCGATGCA CAGACAATCA CACTGGAACG TCAGCCTCAG CCCCCATGGC
1251 TGCTGGAATC ATTGCCCTGG CCCTAGAAGC CAATCCGTTT CTGACCTGGA
1301 GAGACGTGCA GCATGTTATT GTCAGGACTT CCCGTGCGGG ACATTTGAAC
1351 GCTAATGACT GGAAAACCAA TGCTGCTGGT TTTAAGGTGA GCCATCTCTA
1401 TGGATTTGGA CTGATGGATG CCGAAGCCAT GGTGATGGAA GCAGAGAAGT
1451 GGACAACTGT TCCTCAGCAG CACGTGTGTG TGGAAAGCAC AGACCGACAA
1501 ATCAAGACCA TTCGACCAAA CAGTGCAGTG CGCTCCATCT ACAAAGCCTC
1551 AGGCTGCTCG GATAATCCCA ACCATCACGT CAATTACCTG GAGCATGTAG
1601 TTGTGCGTAT TACCATCACA CACCCACGGA GGGGAGACCT GGCCATCTAT
1651 CTGACATCAC CCTCAGGAAC CAGATCCCAG CTCTTGGCCA ACAGGCTCTT
1701 TGATCATTCC ATGGAAGGGT TTAAGAACTG GGAGTTCATG ACTATTCATT
1751 GCTGGGGAGA ACGGGCTGCT GGGGACTGGG TCCTCGAAGT TTATGATACG
1801 CCATCTCAGC TGAGGAACTT CAAGACTCCA GGTAAATTGA AAGAATGGTC
1851 CTTAGTCCTC TATGGCACGT CCGTACAGCC ATACTCCCCA ACCAACGAGT
1901 TTCCCAAAGT GGAACGCTTC CGCTACAGCC GAGTGGAAGA CCCCACAGAT
 
1951 GACTACGGTG CTGAAGATTA TGCAGGTCCC TGTGACCCTG AATGCAGTGA
2001 GGTTGGATGT GACGGGCCAG GACCAGATCA CTGCAGTGAC TGCTTACACT
2051 ACTACTACAA GCTGAAAAAT AACACCAGAA TCTGTGTCTC CAGCTGCCCT
2101 CCTGGCCACT ACCATGCTGA CAAGAAGAGG TGCCGGAAGT GTGCCCCAAA
2151 CTGCGAGTCC TGCTTTGGCA GCCATGGTGA TCAGTGCCTC TCCTGTAAAT
2201 ATGGCTACTT CCTGAATGAA GAAACTAGCA GCTGTGTTAC TCAGTGCCCT
2251 GATGGATCAT ACGAGGATAT CAAGAAAAAT GTCTGTGGGA AATGCAGTGA
2301 GAACTGCAAG GCATGCATTG GATTTCACAA CTGCACAGAG TGCAAGGGCG
2351 GGTTAAGTCT TCAGGGATCC CGCTGTTCGG TCACCTGCGA GGATGGACAG
2401 TTCTTCAATG GTCACGACTG CCAGCCCTGC CATCGCTTCT GTGCTACTTG
2451 CTCTGGGGCC GGAGCAGATG GATGTATTAA CTGCACCGAG GGGTATGTCA
2501 TGGAAGAGGG AAGGTGTGTA CAAAGTTGTA GTGTGAGCTA CTACTTGGAC
2551 CACTCTTCAG AGGGTGGCTA CAAATCCTGC AAGAGATGTG ATAACAGCTG
2601 TTTGACATGC AATGGGCCAG GATTCAAGAA CTGTTCCAGC TGCCCCAGTG
2651 GATATCTTTT AGACTTAGGA ACGTGTCAGA TGGGAGCCAT CTGCAAGGAT
2701 GCTACGGAAG AGTCCTGGGC AGAAGGAGGC TTCT
 but is not able to hybridize under at least moderately stringent conditions to the sequences: 
1 TGGAAGGCAA GGACTGGAAT GAAGCCGTGC CCACTGAAAA GCCATCTTTG
51 GTGAGGAGTC TGCTGCAGGA TCGACGAAAG TGGAAAGTTC AAATCAAAAG
101 AGATGCAACG AGCCAGAATC AACCTTGTCA CTCTTCTTGT AAAACCTGCA
151 ATGGATCTCT CTGCGCTTCA TGTCCCACAG GTATGTACCT GTGGCTGCAG
201 GCCTGTGTTC CTTCCTGTCC CCAAGGCACT TGGCCATCAG TCACCAGTGG
251 CAGCTGTGAA AAGTGTTCCG AGGACTGTGT CTCCTGCTCC GGTGCCGACC
301 TTTGCCAACA GTGCCTGAGC CAGCCGGACA ACACTCTGCT TCTCCATGAG
351 GGCAGGTGCT ACCACAGTTG CCCAGAG; or
1 AGGGAGGCTG AGTTTTACGA GCACACCAAG ACTGCTCTGC TAGTGACCTC
51 TGGCGCCATG CTGTTGCTGC TGCTGGGGGC TGCTGCGGTC GTGTGGCGGA
101 AGTCTCGAAG CAGACCTGTG GCAAAGGGGC GGTACGAAAA GCTGGCAGAA
151 CCTACCGTGT CATACTCCTC CTACAGGAGC AGCTATCTTG ACGAGGACCA
201 GGTGATTGAG TACAGGGACC GGGACTACGA TGAGGACGAT GAGGACGACA
251 TCGTCTATAT GGGCCAAGAT GGCACTGTCT ACCGGAAGTT CAAGTATGGG
301 CTGCTGGATG AGACGGAAGA TGATGAGTTG GAGTACGATG ATGAGAGCTA
351 CTCTTACCAA TAAACAGAGC CCCCTCCCAT CTCAAACCCA CCACCCAC
20. A nucleic acid molecule according to claim 19, wherein the nucleic acid molecule is a cDNA, a genomic DNA, or an RNA.
21. A nucleic acid molecule according to claim 19 or claim 20, wherein the nucleic acid molecule is in the sense or the anti-sense orientation.
22. A nucleic acid molecule according to any one of claims 19 to 21, wherein the nucleic acid molecule is a cDNA.
23. A nucleic acid molecule according to any one of claims 19 to 22, wherein the nucleic acid molecule has a sequence selected from the group consisting of:
   (a) a cDNA molecule having the sequence set out in SEQ. ID. NO: 1, SEQ ID NO: 2;
   (b) a nucleic acid molecule which is able to hybridise under at least moderately stringent conditions to (a); or
   (c) a nucleic acid molecule which has at least 75% sequence identity to (a).
24. A nucleic acid molecule according to claim 23, wherein in (b) the nucleic acid molecule is able to hybridise under stringent conditions to the molecule of (a).
25. A nucleic acid molecule according to claim 23, wherein in (c), the nucleic acid molecule has at least 80%, sequence identity to the molecule of (a).
26. A method according to any one of claims 1 to 18, wherein the proprotein convertase 5/6 is an isoform of proprotein convertase 5/6 which is specifically expressed in implantation sites of the uterus during embryo implantation.
27. A method according to any one of claims 1 to 18, wherein the isoform is encoded by a nucleic acid molecule according to any one of claims 19 to 25.
28. A protein having proprotein convertase 5/6 activity, encoded by the nucleic acid molecule of any one of claims 19 to 25.
29. A pharmaceutical composition comprising a proprotein convertase 5/6 according to claim 28, together with a pharmaceutically acceptable carrier.

## Claims

1. A Proprotein convertase 5/6 (PC 5/6) antagonist for inhibiting fertility in a female mammal.

2. The antagonist of claim 1, wherein fertility is inhibited by inhibiting conversion of a uterus of the female mammal from a non-receptive state to a receptive state.

3. A PC 5/6 antagonist for preventing establishment of a pregnancy.

4. The antagonist of claim 3, wherein establishment is prevented by preventing embryo implantation.

5. The antagonist of claim 1 or claim 3, wherein the antagonist is an antibody or an anti-sense nucleic acid.

6. The antagonist of claim 5, wherein the antibody is a neutralising antibody.

7. The antagonist of claim 5, wherein the anti-sense nucleic acid comprises a nucleic acid that is antisense to SEQ ID NO: 1, SEQ ID NO: 2, or SEQ ID NO: 3.

8. The antagonist of claim 1 or 3, wherein the PC 5/6 is from a mammal species that is different from the mammal being treated.

9. The antagonist of claim 1 or claim 3, wherein the antagonist is combined with a pharmaceutically acceptable carrier.

10. The antagonist of claim 1 or claim 3, wherein the mammal is a human, a companion mammal, a domestic mammal, a zoo mammal, or a feral mammal.

11. A method of screening for antagonists of PC 5/6 activity, comprising assessing the ability of a candidate compound to decrease proprotein convertase 5/6 activity relative to a control.


SEQUENCE LISTING

<110>  Prince Henry's Institute of Medical Research

<120>  Pregnancy-related enzyme activity

<130>  P42984

<140>  AU PR 6730
<141>  2001-07-31

<160>  51

<170>  PatentIn version 3.1

<210>  1
<211>  2994
<212>  DNA
<213>  Mus sp.

<400>  1
gaagttagtt gtgcgcgctg cttagcgcgc gagccagcgg gcgggcgaag gcggcgaagc      60

gtcgggacca tggactggga ctgggggaac cgctgcagcc gcccgggacg gcgggacctg     120

ctgtgcgtgc tggcactgct cgccggctgt ctgctcccgg tatgccggac gcgcgtctac     180

accaaccact gggcagtgaa gatcgccggc ggcttcgcgg aggcagatcg catagccagc     240

aagtacggat tcatcaacgt aggacagatc ggtgcactga aggactacta tcacttctac     300

catagtagga ccattaaaag gtctgttctc tcgagcagag gaacccacag tttcatttca     360

atggaaccaa aggtggagtg gatccaacag caagtggtga aaaaaagaac caagagggat     420

tatgacctca gccatgccca gtcaacctac ttcaatgatc ccaagtggcc aagtatgtgg     480

tacatgcact gcagtgacaa cacccatcct tgccagtcag acatgaatat cgaaggagcc     540

tggaagagag gctacacggg gaagaatatc gtggtgacta tcctggatga cggcatcgag     600

agaacccacc cagatctgat gcaaaactac gatgctctgg caagttgcga tgtgaatggg     660

aatgacttgg acccgatgcc tcgttatgat gcaagcaatg agaacaagca tgggacccgc     720

tgtgccggag aagtggcagc cactgcaaac aactctcact gcactgtcgg gatcgctttc     780

aacgccaaga ttggaggtgt gagaatgctg gatggtgatg tcactgacat ggtggaggca     840

aagtctgtca gctacaaccc acagcatgtg cacatctaca gtgccagctg gggaccagat     900

gacgatggca agactgtgga tgggccagct cccctcaccc ggcaagcctt tgagaatggc     960

gtgagaatgg ggcggagagg ccttggatct gtgtttgtgt gggcatctgg caatggtgga    1020

cggagcaagg atcactgttc ttgtgatggc tataccaaca gcatctatac catctccatc    1080

agcagtacgg ccgaaagtgg aaagaaacct tggtacttgg aagagtgttc atctacactg    1140

gctacaacct acagcagtgg agaatcctat gataagaaaa taatcactac tgatctaagg    1200

cagcgatgca cagacaatca cactggaacg tcagcctcag cccccatggc tgctggaatc 1260

attgccctgg ccctagaagc caatccgttt ctgacctgga gagacgtgca gcatgttatt 1320

gtcaggactt cccgtgcggg acatttgaac gctaatgact ggaaaaccaa tgctgctggt 1380

tttaaggtga gccatctcta tggatttgga ctgatggatg ccgaagccat ggtgatggaa 1440

gcagagaagt ggacaactgt tcctcagcag cacgtgtgtg tggaaagcac agaccgacaa 1500

atcaagacca ttcgaccaaa cagtgcagtg cgctccatct acaaagcctc aggctgctcg 1560

gataatccca accatcacgt caattacctg gagcatgtag ttgtgcgtat taccatcaca 1620

cacccacgga ggggagacct ggccatctat ctgacatcac cctcaggaac cagatcccag 1680

ctcttggcca acaggctctt tgatcattcc atggaagggt ttaagaactg ggagttcatg 1740

actattcatt gctggggaga acgggctgct ggggactggg tcctcgaagt ttatgatacg 1800

ccatctcagc tgaggaactt caagactcca ggtaaattga aagaatggtc cttagtcctc 1860

tatggcacgt ccgtacagcc atactcccca accaacgagt ttcccaaagt ggaacgcttc 1920

cgctacagcc gagtggaaga ccccacagat gactacggtg ctgaagatta tgcaggtccc 1980

tgtgaccctg aatgcagtga ggttggatgt gacgggccag gaccagatca ctgcagtgac 2040

tgcttacact actactacaa gctgaaaaat aacaccagaa tctgtgtctc cagctgccct 2100

cctggccact accatgctga caagaagagg tgccggaagt gtgccccaaa ctgcgagtcc 2160

tgctttggca gccatggtga tcagtgcctc tcctgtaaat atggctactt cctgaatgaa 2220

gaaactagca gctgtgttac tcagtgccct gatggatcat acgaggatat caagaaaaat 2280

gtctgtggga aatgcagtga gaactgcaag gcatgcattg gatttcacaa ctgcacagag 2340

tgcaagggcg ggttaagtct tcaggatccc cgctgttcgg tcacctgcga ggatggacag 2400

ttcttcaatg gtcacgactg ccagccctgc catcgcttct gtgctacttg ctctggggcc 2460

ggagcagatg gatgtattaa ctgcaccgag gggtatgtca tggaagaggg aaggtgtgta 2520

caaagttgta gtgtgagcta ctacttggac cactcttcag agggtggcta caaatcctgc 2580

aagagatgtg ataacagctg tttgacatgc aatgggccag gattcaagaa ctgttccagc 2640

tgccccagtg gatatctttt agacttagga acgtgtcaga tgggagccat ctgcaaggat 2700

gctacggaag agtcctgggc agaaggaggc ttctgtatgc ttgtgaaaaa gaacaatctc 2760

tgtcagcgaa aagttctaca acagctgtgc tgcaaaacat gtacattcca aggctgagca 2820

gccgccttcc atccctgttc ctgtagacgt atagattatt ccatattatt aaagaaaaaa 2880

aaagccaaaa aactgcaagc cacctctctc tttcttgcta tctttttttt ctctctctct 2940

ctatctcaag tttgtgcagg gagatggtga actgttttgt cagaacttaa atag 2994

<210> 2
<211> 5208
<212> DNA
<213> Mus sp.

<400> 2
aacagcatct ataccatctc catcagcagt acggccgaaa gtggaaagaa accttggtac 60

ttggaagagt gttcatctac actggctaca acctacagca gtggagaatc ctatgataag 120

aaaataatca ctactgatct aaggcagcga tgcacagaca atcacactgg aacgtcagcc 180

tcagccccca tggctgctgg aatcattgcc ctggccctag aagccaatcc gtttctgacc 240

tggagagacg tgcagcatgt tattgtcagg acttcccgtg cgggacattt gaacgctaat 300

gactggaaaa ccaatgctgc tggttttaag gtgagccatc tctatggatt tggactgatg 360

gatgccgaag ccatggtgat ggaagcagag aagtggacaa ctgttcctca gcagcacgtg 420

tgtgtggaaa gcacagaccg acaaatcaag accattcgac caaacagtgc agtgcgctcc 480

atctacaaag cctcaggctg ctcggataat cccaaccatc acgtcaatta cctggagcat 540

gtagttgtgc gtattaccat cacacaccca cggaggggag acctggccat ctatctgaca 600

tcaccctcag gaaccagatc ccagctcttg gccaacaggc tctttgatca ttccatggaa 660

gggtttaaga actgggagtt catgactatt cattgctggg gagaacgggc tgctggggac 720

tgggtcctcg aagtttatga tacgccatct cagctgagga acttcaagac tccaggtaaa 780

ttgaaagaat ggtccttagt cctctatggc acgtccgtac agccatactc cccaaccaac 840

gagtttccca aagtggaacg cttccgctac agccgagtgg aagaccccac agatgactac 900

ggtgctgaag attatgcagg tccctgtgac cctgaatgca gtgaggttgg atgtgacggg 960

ccaggaccag atcactgcag tgactgctta cactactact acaagctgaa aaataacacc 1020

agaatctgtg tctccagctg ccctcctggc cactaccatg ctgacaagaa gaggtgccgg 1080

aagtgtgccc caaactgcga gtcctgcttt ggcagccatg gtgatcagtg cctctcctgt 1140

aaatatggct acttcctgaa tgaagaaact agcagctgtg ttactcagtg ccctgatgga 1200

tcatacgagg atatcaagaa aaatgtctgt gggaaatgca gtgagaactg caaggcatgc 1260

attggatttc acaactgcac agagtgcaag ggcgggttaa gtcttcaggg atcccgctgt 1320

tcggtcacct gcgaggatgg acagttcttc aatggtcacg actgccagcc ctgccatcgc 1380

ttctgtgcta cttgctctgg ggccggagca gatggatgta ttaactgcac cgaggggtat 1440

gtcatggaag agggaaggtg tgtacaaagt tgtagtgtga gctactactt ggaccactct 1500

tcagagggtg gctacaaatc ctgcaagaga tgtgataaca gctgtttgac atgcaatggg 1560

ccaggattca agaactgttc cagctgcccc agtggatatc ttttagactt aggaacgtgt 1620

cagatgggag ccatctgcaa ggatggagaa tacattgatg accaaggcca ctgccaaacc 1680

tgtgaagcct catgtgccaa gtgctgggga ccaactcagg aagactgtat cagctgcccc 1740
gtaacaaggg tcttggatga tggtcgctgt gttatgaact gtccttcctg gaagttcgaa 1800
tttaagaagc aatgccatcc ctgccactac acttgtcaag gatgccaagg tagtggccct 1860
tccaactgca cctcctgtag agcagacaag catggtcagg agcgcttcct gtaccacggg 1920
gaatgtctgg agaactgccc tgtgggccat tatcctgcca agggacacac ctgcctaccc 1980
tgcccagaca actgtgagct ttgctacaac ccacacatct gcagtcgatg catgagtggc 2040
tatgtcatca ttccccccaa ccacacctgc cagaagctgg agtgcagaca aggtgaattc 2100
caggattctg agtatgaaga atgcatgccc tgtgaggaag gatgtctggg atgcactgag 2160
gatgatccag gagcctgtac ctcgtgtgct acaggatatt acatgtttga gcggcattgc 2220
tataaagcct gtcctgagaa gaccttcggt gtgaaatggg aatgcagggc ctgtggtact 2280
aactgtggca gctgcgacca acatgagtgc tactggtgtg aggagggctt ctttctctcc 2340
ggtggcagct gtgtgcagga ttgtggccct ggcttccatg gagaccaaga gttgggagaa 2400
tgcaagccct gccaccgagc ctgtgagact tgcactggct cgggctacaa ccaatgcagc 2460
agctgtcaag aagggttgca gctatggcat gggacgtgtc tctggtccac ctggcctcag 2520
gtggaaggca aggactggaa tgaagccgtg cccactgaaa agccatcttt ggtgaggagt 2580
ctgctgcagg atcgacgaaa gtggaaagtt caaatcaaaa gagatgcaac gagccagaat 2640
caaccttgtc actcttcttg taaaacctgc aatggatctc tctgcgcttc atgtcccaca 2700
ggtatgtacc tgtggctgca ggcctgtgtt ccttcctgtc cccaaggcac ttggccatca 2760
gtcaccagtg gcagctgtga aaagtgttcc gaggactgtg tctcctgctc cggtgccgac 2820
ctttgccaac agtgcctgag ccagccggac aacactctgc ttctccatga gggcaggtgc 2880
taccacagtt gcccagaggg cttttatgca aaagatggtg tttgtgaaca ctgtagttcc 2940
ccttgcaaaa catgcgaagg aaatgccacc agctgtaact cttgtgaagg agacttcgtc 3000
ctagaccatg gggtgtgctg gaaaacttgc cctgaaaagc acgtggccgt ggaaggagtc 3060
tgcaagcact gtccagagag gtgccaggac tgcatccacg agaaaacttg caaagagtgc 3120
atgcctgact tctttctata caatgacatg tgccatcgtt cctgtcccaa gagcttctac 3180
cctgacatgc gccagtgtgt cccctgccac aaaaactgtc tggagtgcaa tggccccaag 3240
gaagatgact gtaaggtctg tgctgatact tctaaggctc tccacaatgg attgtgcttg 3300
gatgagtgcc ctgagggaac ctacaaagaa gaagagaatg atgaatgcag agattgcccc 3360
gagtcctgcc tgatctgctc atcagcttgg acctgcctgg cctgtcggga aggcttcaca 3420
gtagtgcatg atgtctgcac ggcacccaag gagtgtgcag ccgtcgagta ctgggatgag 3480

ggctcccaca ggtgccagcc gtgtcacaag aaatgctccc gctgctcagg gccttctgag 3540
gaccagtgct atacctgtcc cagagagact tttcttctca atacaacctg tgtgaaagag 3600
tgcccagagg gctaccacac tgacaaggac agccagcagt gtgtgctctg ccacagctct 3660
tgcaggacct gcgaaggacc acacagcatg cagtgcctct cctgccgacc tggctggttt 3720
caactgggca aggagtgctt gctgcaatgc agggatggat attatggaga aagcaccagc 3780
ggtaggtgtg agaagtgtga caagagctgc aagtcatgcc gaggtccacg gcccacagac 3840
tgccagtctt gcgatacatt tttctttctc ctacgctcca agggacagtg ccaccgcgct 3900
tgccctgagc actactatgc agaccaacac gcccagacct gtgagaggtg ccaccccact 3960
tgtgacaagt gcagcggaaa ggaggcgtgg agttgcttgt cctgtgtgtg gagctaccac 4020
cttctgaaag gaatctgcat cccagaatgt atcgtagggg aatacagaga gggaaaggga 4080
gaaaacttta actgtaaaaa atgccacgag agctgcatgg aatgcaaggg tccaggcagc 4140
aagaactgca ccgggtgctc agctggcctg ctgctggaca tggacgacaa ccgctgcctc 4200
cattgctgca atgcctccca ctcccgcaga tcccaggact gctgcgactg ccagagttcc 4260
acagatgagt gcatccttcc agccagggag gctgagtttt acgagcacac caagactgct 4320
ctgctagtga cctctggcgc catgctgttg ctgctgctgg gggctgctgc ggtcgtgtgg 4380
cggaagtctc gaagcagacc tgtggcaaag ggcggtacgg aaaagctggc agaacctacc 4440
gtgtcatact cctcctacag gagcagctat cttgacgagg accaggtgat tgagtacagg 4500
gaccgggact acgatgagga cgatgaggac gacatcgtct atatgggcca agatggcact 4560
gtctaccgga agttcaagta tgggctgctg gatgagacgg aagatgatga gttggagtac 4620
gatgatgaga gctactctta ccaataaaca gagccccctc ccatctcaaa cccaccaccc 4680
accacccacc tcccatccct tcctggtccc ttcctggctc tcaatcctgc tgggagctac 4740
caatgcattg gctgaggtct gagtgtttgc ttttgtcctt accttgcatc caacccgaat 4800
atgtagggat gctggagtct gctgtgctgc ttttcattag ctgagtgcaa tgaacagatc 4860
ctgcaaggag agaattcgaa ggcattttcc ccgagggtat gtcaagggta ttaaatgcag 4920
gaatctaaaa gaactaagat ctgttgaaca ctctaatgtg atttttaaag tggatggaga 4980
agaggacctg taagggttct gctttcaaac tgtagttgga gactcactct catccccaca 5040
actgctacct tctagaattc tgatatttga ctaaatagag ggagatggga gtactagaga 5100
gttgttggag ctggggtggg aggtctctcc actttcaatc gtggaaagaa agaaagaaag 5160
aaagaaagaa agaaagaaag aaagaaagaa agaaagaaag aaagaaag 5208

<210> 3
<211> 2766

<212>  DNA
<213>  Homo sapiens

<400>  3
agcgtcggga ccatggattg ggattggggg aaccgctgca gccgcccggg acggcgggac      60

ctgctgtgcg tgctggcact gctcgccggc tgtctgctcc cggtatgccg gacgcgcgtc     120

tacaccaacc actgggcagt gaagatcgcc ggcggcttcg cggaggcaga tcgcatagcc     180

agcaagtacg gattcatcaa cgtaggacag atcggtgcac tgaaggacta ctatcacttc     240

taccatagta ggaccattaa aaggtctgtt ctctcgagca gaggaaccca cagtttcatt     300

tcaatggaac caaaggtgga gtggatccaa cagcaagtgg tgaaaaaaag aaccaagagg     360

gattatgacc tcagccatgc ccagtcaacc tacttcaatg atcccaagtg gccaagtatg     420

tggtacatgc actgtagcga caatacacat ccctgccagt ctgacatgaa tatcgaagga     480

gcctggaaga gaggctacac gggaaagaac attgtggtca ctatcctgga tgacggaatt     540

gagagaaccc atccagatct gatgcaaaac tacgatgctc tggcaagttg cgacgtgaat     600

gggaatgact tggacccaat gcctcgttat gatgcaagca acgagaacaa gcatgggact     660

cgctgtgctg gagaagtggc agccgctgca aacaattcgc actgcacagt cggaattgct     720

ttcaacgcca agatcggagg agtgcgaatg ctggacggag atgtcacgga catggttgaa     780

gcaaaatcag ttagcttcaa cccccagcac gtgcacattt acagcgccag ctggggcccg     840

gatgatgatg gcaagactgt ggacggacca gcccccctca cccggcaagc ctttgaaaac     900

ggcgttagaa tggggcggag aggcctcggc tctgtgtttg tttgggcatc tggaaatggt     960

ggaaggagca aagaccactg ctcctgtgat ggctacacca acagcatcta caccatctcc    1020

atcagcagca ctgcagaaag cggaaagaaa ccttggtacc tggaagagtg ttcatccacg    1080

ctggccacaa cctacagcag cggggagtcc tacgataaga aaatcatcac tacagatctg    1140

aggcagcgtt gcacggacaa ccacactggg acgtcagcct cagcccccat ggctgcaggc    1200

atcattgcgc tggccctgga agccaatccg tttctgacct ggagagacgt acagcatgtt    1260

attgtcagga cttcccgtgc gggacatttg aacgctaatg actggaaaac caatgctgct    1320

ggttttaagg tgagccatct ttatggattt ggactgatgg acgcagaagc catggtgatg    1380

gaggcagaga agtggaccac cgttccccgg cagcacgtgt gtgtggagag cacagaccga    1440

caaatcaaga caatccgccc taacagtgca gtgcgctcca tctacaaagc ttcaggctgc    1500

tcggataacc ccaaccgcca tgtcaactac ctggagcacg tcgttgtgcg catcaccatc    1560

acccacccca ggagaggaga cctggccatc tacctgacct cgccctctgg aactaggtct    1620

cagcttttgg ccaacaggct atttgatcac tccatggaag gattcaaaaa ctgggagttc    1680

atgaccattc attgctgggg agaaagagct gctggtgact gggtccttga agtttatgat    1740

actccctctc agctaaggaa ctttaagact ccaggtaaat tgaaagaatg gtctttggtc 1800

ctctacggca cctccgtgcg gccatattca ccaaccaatg aatttccgaa agtggaacgg 1860

ttccgctata gccgagttga agaccccaca gacgactatg gcacagagga ttatgcaggt 1920

ccctgcgacc ctgagtgcag tgaggttggc tgtgacgggc caggaccaga ccactgcaat 1980

gactgtttgc actactacta caagctgaaa aacaatacca ggatctgtgt ctccagctgc 2040

ccccctggcc actaccacgc cgacaagaag cgctgcagga agtgtgcccc caactgtgag 2100

tcctgctttg ggagccatgg tgaccaatgc atgtcctgca aatatggata ctttctgaat 2160

gaagaaacca acagctgtgt tactcactgc cctgatgggt catatcagga taccaagaaa 2220

aatctttgcc ggaaatgcag tgaaaactgc aagacatgta ctgaattcca taactgtaca 2280

gaatgtaggg atgggttaag cctgcaggga tcccggtgct ctgtctcctg tgaagatgga 2340

cggtatttca acggccagga ctgccagccc tgccaccgct tctgcgccac ttgtgctggg 2400

gcaggagctg atgggtgcat taactgcaca gagggctact tcatggagga tgggagatgc 2460

gtgcagagct gtagtatcag ctattacttt gaccactctt cagagaatgg atacaaatcc 2520

tgcaaaaaat gtgatatcag ttgtttgacg tgcaatggcc caggattcaa gaactgtaca 2580

agctgcccta gtgggtatct cttagactta ggaatgtgtc aaatgggagc catttgcaag 2640

gatgcaacgg aagagtcctg ggcggaagga ggcttctgta tgcttgtgaa aaagaacaat 2700

ctgtgccaac ggaaggttct tcaacaactt tgctgcaaaa catgtacatt ccaaggctga 2760

gcagcc 2766

<210> 4
<211> 2734
<212> DNA
<213> Mus sp.

<400> 4
gaagttagtt gtgcgcgctg cttagcgcgc gagccagcgg gcgggcgaag gcggcgaagc 60

gtcgggacca tggactggga ctgggggaac cgctgcagcc gcccgggacg gcgggacctg 120

ctgtgcgtgc tggcactgct cgccggctgt ctgctcccgg tatgccggac gcgcgtctac 180

accaaccact gggcagtgaa gatcgccggc ggcttcgcgg aggcagatcg catagccagc 240

aagtacggat tcatcaacgt aggacagatc ggtgcactga aggactacta tcacttctac 300

catagtagga ccattaaaag gtctgttctc tcgagcagag gaacccacag tttcatttca 360

atggaaccaa aggtggagtg gatccaacag caagtggtga aaaaaagaac caagagggat 420

tatgacctca gccatgccca gtcaacctac ttcaatgatc ccaagtggcc aagtatgtgg 480

tacatgcact gcagtgacaa cacccatcct tgccagtcag acatgaatat cgaaggagcc 540

tggaagagag gctacacggg gaagaatatc gtggtgacta tcctggatga cggcatcgag 600
agaacccacc cagatctgat gcaaaactac gatgctctgg caagttgcga tgtgaatggg 660
aatgacttgg acccgatgcc tcgttatgat gcaagcaatg agaacaagca tgggacccgc 720
tgtgccggag aagtggcagc cactgcaaac aactctcact gcactgtcgg gatcgctttc 780
aacgccaaga ttggaggtgt gagaatgctg gatggtgatg tcactgacat ggtggaggca 840
aagtctgtca gctacaaccc acagcatgtg cacatctaca gtgccagctg gggaccagat 900
gacgatggca agactgtgga tgggccagct cccctcaccc ggcaagcctt tgagaatggc 960
gtgagaatgg ggcggagagg ccttggatct gtgtttgtgt gggcatctgg caatggtgga 1020
cggagcaagg atcactgttc ttgtgatggc tataccaaca gcatctatac catctccatc 1080
agcagtacgg ccgaaagtgg aaagaaacct tggtacttgg aagagtgttc atctacactg 1140
gctacaacct acagcagtgg agaatcctat gataagaaaa taatcactac tgatctaagg 1200
cagcgatgca cagacaatca cactggaacg tcagcctcag cccccatggc tgctggaatc 1260
attgccctgg ccctagaagc caatccgttt ctgacctgga gagacgtgca gcatgttatt 1320
gtcaggactt cccgtgcggg acatttgaac gctaatgact ggaaaaccaa tgctgctggt 1380
tttaaggtga gccatctcta tggatttgga ctgatggatg ccgaagccat ggtgatggaa 1440
gcagagaagt ggacaactgt tcctcagcag cacgtgtgtg tggaaagcac agaccgacaa 1500
atcaagacca ttcgaccaaa cagtgcagtg cgctccatct acaaagcctc aggctgctcg 1560
gataatccca accatcacgt caattacctg gagcatgtag ttgtgcgtat taccatcaca 1620
cacccacgga ggggagacct ggccatctat ctgacatcac cctcaggaac cagatcccag 1680
ctcttggcca acaggctctt tgatcattcc atggaagggt ttaagaactg ggagttcatg 1740
actattcatt gctggggaga acgggctgct ggggactggg tcctcgaagt ttatgatacg 1800
ccatctcagc tgaggaactt caagactcca ggtaaattga aagaatggtc cttagtcctc 1860
tatggcacgt ccgtacagcc atactcccca accaacgagt ttcccaaagt ggaacgcttc 1920
cgctacagcc gagtggaaga ccccacagat gactacggtg ctgaagatta tgcaggtccc 1980
tgtgaccctg aatgcagtga ggttggatgt gacgggccag gaccagatca ctgcagtgac 2040
tgcttacact actactacaa gctgaaaaat aacaccagaa tctgtgtctc cagctgccct 2100
cctggccact accatgctga caagaagagg tgccggaagt gtgccccaaa ctgcgagtcc 2160
tgctttggca gccatggtga tcagtgcctc tcctgtaaat atggctactt cctgaatgaa 2220
gaaactagca gctgtgttac tcagtgccct gatggatcat acgaggatat caagaaaaat 2280
gtctgtggga aatgcagtga gaactgcaag gcatgcattg gatttcacaa ctgcacagag 2340

tgcaagggcg ggttaagtct tcagggatcc cgctgttcgg tcacctgcga ggatggacag 2400

ttcttcaatg gtcacgactg ccagccctgc catcgcttct gtgctacttg ctctggggcc 2460

ggagcagatg gatgtattaa ctgcaccgag gggtatgtca tggaagaggg aaggtgtgta 2520

caaagttgta gtgtgagcta ctacttggac cactcttcag agggtggcta caaatcctgc 2580

aagagatgtg ataacagctg tttgacatgc aatgggccag gattcaagaa ctgttccagc 2640

tgccccagtg gatatctttt agacttagga acgtgtcaga tgggagccat ctgcaaggat 2700

gctacggaag agtcctgggc agaaggaggc ttct 2734

<210> 5
<211> 377
<212> DNA
<213> Mus sp.

<400> 5
tggaaggcaa ggactggaat gaagccgtgc ccactgaaaa gccatctttg gtgaggagtc 60

tgctgcagga tcgacgaaag tggaaagttc aaatcaaaag agatgcaacg agccagaatc 120

aaccttgtca ctcttcttgt aaaacctgca atggatctct ctgcgcttca tgtcccacag 180

gtatgtacct gtggctgcag gcctgtgttc cttcctgtcc ccaaggcact tggccatcag 240

tcaccagtgg cagctgtgaa aagtgttccg aggactgtgt ctcctgctcc ggtgccgacc 300

tttgccaaca gtgcctgagc cagccggaca acactctgct tctccatgag ggcaggtgct 360

accacagttg cccagag 377

<210> 6
<211> 398
<212> DNA
<213> Mus sp.

<400> 6
agggaggctg agttttacga gcacaccaag actgctctgc tagtgacctc tggcgccatg 60

ctgttgctgc tgctgggggc tgctgcggtc gtgtggcgga agtctcgaag cagacctgtg 120

gcaaaggggc ggtacgaaaa gctggcagaa cctaccgtgt catactcctc ctacaggagc 180

agctatcttg acgaggacca ggtgattgag tacagggacc gggactacga tgaggacgat 240

gaggacgaca tcgtctatat gggccaagat ggcactgtct accggaagtt caagtatggg 300

ctgctggatg agacggaaga tgatgagttg gagtacgatg atgagagcta ctcttaccaa 360

taaacagagc cccctcccat ctcaaaccca ccacccac 398

<210> 7
<211> 14
<212> DNA
<213> Artificial Sequence

<220>
<223>  3' primer

<400>  7
tttttttttt ttva                                                          14


<210>  8
<211>  14
<212>  DNA
<213>  Artificial Sequence

<220>
<223>  3' primer

<400>  8
tttttttttt ttvc                                                          14


<210>  9
<211>  14
<212>  DNA
<213>  Artificial Sequence

<220>
<223>  3' primer

<400>  9
tttttttttt ttvg                                                          14


<210>  10
<211>  14
<212>  DNA
<213>  Artificial Sequence

<220>
<223>  3' primer

<400>  10
tttttttttt ttvt                                                          14


<210>  11
<211>  10
<212>  DNA
<213>  Artificial Sequence

<220>
<223>  5' primer

<400>  11
caggcccttc                                                               10


<210>  12
<211>  10
<212>  DNA
<213>  Artificial Sequence

<220>
<223>  5' primer

<400>  12
tgccgagctg                                                              10


<210>  13
<211>  10
<212>  DNA
<213>  Artificial Sequence

<220>
<223>  5' primer

<400>  13
agtcagccac                                                              10


<210>  14
<211>  10
<212>  DNA
<213>  Artificial Sequence

<220>
<223>  5' primer

<400>  14
aatcgggctg                                                              10


<210>  15
<211>  10
<212>  DNA
<213>  Artificial Sequence

<220>
<223>  5' primer

<400>  15
aggggtcttg                                                              10


<210>  16
<211>  10
<212>  DNA
<213>  Artificial Sequence

<220>
<223>  5' primer

<400>  16
ggtccctgac                                                              10


<210>  17
<211>  10
<212>  DNA
<213>  Artificial Sequence

<220>

<223>  5' primer

<400>  17
gaaacgggtg                                                              10


<210>  18
<211>  10
<212>  DNA
<213>  Artificial Sequence

<220>
<223>  5' primer

<400>  18
gtgacgtagg                                                              10


<210>  19
<211>  10
<212>  DNA
<213>  Artificial Sequence

<220>
<223>  5' primer

<400>  19
gggtaacgcc                                                              10


<210>  20
<211>  10
<212>  DNA
<213>  Artificial Sequence

<220>
<223>  5' primer

<400>  20
gtgatcgcag                                                              10


<210>  21
<211>  10
<212>  DNA
<213>  Artificial Sequence

<220>
<223>  5' primer

<400>  21
caatcgccgt                                                              10


<210>  22
<211>  10
<212>  DNA
<213>  Artificial Sequence

<220>
<223>  5' primer

<400>  22
tcggcgatag                                                              10


<210>  23
<211>  10
<212>  DNA
<213>  Artificial Sequence

<220>
<223>  5' primer

<400>  23
cagcacccac                                                              10


<210>  24
<211>  10
<212>  DNA
<213>  Artificial Sequence

<220>
<223>  5' primer

<400>  24
tctgtgctgg                                                              10


<210>  25
<211>  10
<212>  DNA
<213>  Artificial Sequence

<220>
<223>  5' primer

<400>  25
ttccgaaccc                                                              10


<210>  26
<211>  10
<212>  DNA
<213>  Artificial Sequence

<220>
<223>  5' primer

<400>  26
agccagcgaa                                                              10


<210>  27
<211>  10
<212>  DNA
<213>  Artificial Sequence

<220>
<223>  5' primer

<400>  27
gaccgcttgt                                                              10


<210>  28
<211>  10
<212>  DNA
<213>  Artificial Sequence

<220>
<223>  5' primer

<400>  28
aggtgaccgt                                                              10


<210>  29
<211>  10
<212>  DNA
<213>  Artificial Sequence

<220>
<223>  5' primer

<400>  29
caaacgtcgg                                                              10


<210>  30
<211>  10
<212>  DNA
<213>  Artificial Sequence

<220>
<223>  5' primer

<400>  30
gttgcgatcc                                                              10


<210>  31
<211>  21
<212>  DNA
<213>  Artificial Sequence

<220>
<223>  primer

<400>  31
gttagttgtg cgcgctgctt a                                                 21


<210>  32
<211>  16
<212>  DNA
<213>  Artificial Sequence

<220>
<223>  primer

<400>  32

gcgcgtccgg catacc                                                    16

<210>  33
<211>  20
<212>  DNA
<213>  Artificial Sequence

<220>
<223>  primer

<400>  33
cacccatcct tgccagtcag                                                20

<210>  34
<211>  21
<212>  DNA
<213>  Artificial Sequence

<220>
<223>  primer

<400>  34
catccacagt cttgccatcg t                                              21

<210>  35
<211>  21
<212>  DNA
<213>  Artificial Sequence

<220>
<223>  primer

<400>  35
gatcggtgca ctgaaggact a                                              21

<210>  36
<211>  20
<212>  DNA
<213>  Artificial Sequence

<220>
<223>  primer

<400>  36
ctggcagcat gttattgtca                                                20

<210>  37
<211>  21
<212>  DNA
<213>  Artificial Sequence

<220>
<223>  primer

<400>  37
cgtagtcatc tgtggggtct t                                              21

<210>  38
<211>  20
<212>  DNA
<213>  Artificial Sequence

<220>
<223>  primer

<400>  38
ccactaccat gctgacaaga                                                    20


<210>  39
<211>  21
<212>  DNA
<213>  Artificial Sequence

<220>
<223>  primer

<400>  39
agaacttttc gctgacagag a                                                  21


<210>  40
<211>  21
<212>  DNA
<213>  Artificial Sequence

<220>
<223>  primer

<400>  40
gtatgcttgt gaaaaagaac a                                                  21


<210>  41
<211>  21
<212>  DNA
<213>  Artificial Sequence

<220>
<223>  primer

<400>  41
ccctgcacaa acttgagata g                                                  21


<210>  42
<211>  21
<212>  DNA
<213>  Artificial Sequence

<220>
<223>  primer

<400>  42
tggaaggcaa ggactggaat g                                                  21

<210> 43
<211> 21
<212> DNA
<213> Artificial Sequence

<220>
<223> primer

<400> 43
ctctgggcaa cttgtgtagc a 21


<210> 44
<211> 21
<212> DNA
<213> Artificial Sequence

<220>
<223> primer

<400> 44
agggaggctg agttttacga g 21


<210> 45
<211> 18
<212> DNA
<213> Artificial Sequence

<220>
<223> primer

<400> 45
gtgggtggtg ggtttgag 18


<210> 46
<211> 158
<212> DNA
<213> Mus sp.

<220>
<221> primer_bind
<222> (1)..(10)
<223> primer used during DDPCR amplification


<220>
<221> primer_bind
<222> (145)..(158)
<223> primer used during DDPCR amplification


<400> 46
   gtgacgtagg acagatcggt gcactgaagg actactatca cttctaccat agtaggacca 60
   ttaaaaggtc tgttctctcg agcagaggaa cccacagttt catttcaatg gaaccaaagg 120
   tggagtggat ccaacagcaa gtggtgaaaa aaaaaaaa 158

<210>  47
<211>  33
<212>  DNA
<213>  Artificial

<220>
<221>  misc_feature
<222>  (1)..(10)
<223>  primer of OPA-8


<400>  47
gtgacgtagg acagatcggt gcactgaagg act                                    33


<210>  48
<211>  65
<212>  DNA
<213>  Artificial

<220>
<221>  misc_feature
<222>  (52)..(65)
<223>  reverse complementary seq of T12MA


<400>  48
acagtttcat ttcaatggaa ccaaaggtgg agtggatcca acagcaagtg gtgaaaaaaa      60

aaaaa                                                                   65


<210>  49
<211>  60
<212>  DNA
<213>  Mus sp.

<400>  49
atcgcatagc cagcaagtac ggattcatca acgtaggaca gatcggtgca ctgaaggact      60


<210>  50
<211>  65
<212>  DNA
<213>  Mus sp.

<400>  50
acagtttcat ttcaatggaa ccaaaggtgg agtggatcca acagcaagtg gtgaaaaaaa      60

gaacc                                                                   65


<210>  51
<211>  18
<212>  DNA
<213>  Artificial

<400>  51
ccagcattcg cactcctc                                                     18